# EUROPEAN PATENT SPECIFICATION

(11) **EP 1 578 387 B1**
(45) Date of publication and mention of the grant of the patent: **13.08.2008**
(21) Application number: 03808295.4
(22) Date of filing: 08.12.2003
(51) Int. Cl.: A61K 8/898, A61K 8/37, A61Q 1/00, A61Q 19/00

(54) **TRANSPARENT OR TRANSLUCENT CARE AND/OR MAKE-UP COSMETIC COMPOSITION STRUCTURED WITH SILICONE POLYMERS**
DURCHSICHTIGE ODER DURCHSCHEINENDE KOSMETISCHE ZUSAMMENSETZUNG STRUKTURIERT MIT SILIKONPOLYMEREN
COMPOSITION COSMETIQUE DE MAQUILLAGE ET/OU DE SOINS, TRANSPARENTE OU TRANSLUCIDE, A BASE DE POLYMERES DE SILICONE

(30) Priority: 17.12.2002 FR 0216039; 09.01.2003 US 438770 P
(43) Date of publication of application: 28.09.2005
(73) Proprietor: L'ORÉAL, 75008 Paris (FR)
(72) Inventor: BLIN, Xavier, F-75015 Paris (FR); FERRARI, Véronique, F-94700 Maisons-Alfort (FR)
(74) Representative: Chantraine, Sylvie Hélène
(86) International application number: PCT/EP2003/015024
(87) International publication number: WO 2004/054524

(56) References cited:
- EP-A- 0 594 285
- EP-A- 1 266 647
- US-A- 5 874 069
- US-A- 6 051 216
- US-A1- 2002 051 758

## Description

### Technical field

The present invention relates to a care and/or make-up cosmetic composition for the skin, including the scalp, and/or the lips of human beings, containing a liquid fatty phase comprising at least one ester silicone oil, gelled with a particular polymer, the said composition being transparent, translucent and provided in particular in the form of a cast make-up product, in particular as a make-up stick such as a lipstick, whose application leads to a deposit which is also transparent, translucent, nonsticky and tranfer-free.

A care cosmetic composition is a composition which comprises at least one active compound for treating wrinkles, for moisturizing the skin and the lips, for protecting the skin, the lips and superficial body growths from ultraviolet rays, for treating acne and/or for acting as self-tanning agent.

The invention relates more particularly to cosmetic and dermatological compositions which are transparent and translucent, which may be coloured, and the deposits of which are also translucent and transparent, such as make-up products exhibiting non-stick and non-transfer properties.

### Prior state of the art

In cosmetic or dermatological products, it is common to find a structured, namely gelled and/or rigidified, liquid fatty phase; this is in particular the case in solid compositions, balms and lipsticks, eyeshadows, concealer products and foundations which have been cast. This structuring is obtained with the aid of waxes or fillers. Unfortunately, waxes and fillers have the drawback of rendering the composition opaque and tend to mattify the composition due to the pressure of crystals, which is not always desirable in particular for a lipstick or an eyeshadow.

The expression liquid fatty phase is understood to mean, for the purposes of the application, a fatty phase which is liquid at room temperature (25°C) and atmospheric pressure (760 mmHg), which is composed of one or more fatty substances which are liquid at room temperature, which are also called oils, which are compatible with each other and which comprise at least one ester oil.

The expression structured liquid fatty phase is understood to mean a rigidified or gelled liquid fatty phase.

The expression rigidified liquid fatty phase for the purposes of the application is understood to mean that this phase does not run under its own weight when a silicone polyamide is added thereto.

The expression gelled liquid fatty phase for the purposes of the application is understood to mean that the viscosity of this phase is increased because of the addition of silicone polyamide to this fatty phase.

The document EP-A-1 068 856 [1] describes solid cosmetic compositions, with no wax,-containing a liquid fatty phase structured with a polymer, in which the fatty phase is mainly a non-silicone oil.

The document WO-A-01/97758 [2] describes cosmetic compositions based on polyamide resins comprising a gelling agent chosen from esters and amides of N-acylamino acids and mixtures thereof. The composition also comprises a solvent for the polyamide resin which may be chosen from unsaturated and saturated fatty alcohols, fatty and/or aromatic carboxylic acid esters, ethoxylated and/or propoxylated alcohols and acids, silicones, mineral oils and branched-chain hydrocarbons; preferably, fatty acid esters, fatty alcohols, mineral oils, branched hydrocarbons and mixtures thereof.

The fatty phases of cosmetic compositions often make use of silicone oils. Indeed, the use of fatty phases based on silicone oils makes it possible currently to obtain cosmetic compositions having a long staying power when the oils are only slightly volatile or are non-volatile, namely a good staying power in particular of the colour over time (unchanging, unfading), and transfer-free compositions when the silicone oils are volatile, not forming a deposit on a support such as a glass, a cup, a fabric or a cigarette, placed in contact with the film of make-up.

Currently, the use of silicone oils in cosmetics is limited by the small number of molecules which can gel these media and thus give compositions which exist in solid form such as lipsticks or cast foundations for example.

The use of cosmetic compositions whose fatty phase is predominantly siliconized leads, in most cases, to problems of compatibility with the ingredients conventionally used in cosmetics.

In the documents WO-A-97/36573[3], US-A-5 874 069 [4], US-A-5 919 441 [5], US-A-6 051 216 [6], WO-A-02/17870 [7], and WO-A-02/17871 [8], EP-A-1 177 784 [9], WO-A-99/06473 [13], and US-A-6 353 076 [14], which is a division of US-A-6 051 216 cosmetic compositions such as deodorant sticks or gels, comprising a silicone oily phase gelled with a polysiloxane- and polyamide-based wax, or with a polymer containing siloxane groups and groups capable of hydrogen interactions, have been prepared.

More particularly, the document WO-A-97 36573 [3] describes the combination of a fluid silicone solvent and thickening agents which are a wax containing silicone groups, and a polyamide which may contain silicone groups.

The document US-A-5 874 069 [4] is close to the document WO-A-97 36573 [3], but it requires obtaining a solid system by passing from a clear and translucent system, and it gives details on the nature of the wax and its monomeric, in particular siloxane, units.

The document US-A-5 919 441 [5] describes a system containing a fluid with at least one silicone and a polymer with siloxane groups and H bond containing groups, the said polymer being a fluid at room temperature and being soluble in the fluid so as to obtain a clear and translucent solution.

The document US-A-6 051 216 [6] relates to the combination of a silicone polyamide with a fluid silicone and, optionally, an additional solvent.

In WO-A-02/17870 [7], it is envisaged to add to the composition another gelling agent, but the quantities added should be low, for example less than 0.5% in the case of hydroxystearic acid, in order to preserve the clarity of the product.

In WO-A-02/17871 [8], it is also envisaged to use a second gelling agent with the silicone polymer in a quantity representing 0.5 to 2% by weight of the composition, and a solvent system comprising a non-silicone organic compound, a volatile silicone and optionally another silicone.

The document EP-A-1 177 784 [9] illustrates a deodorant composition comprising a liquid phase containing, for example, a volatile silicone and optionally a non-volatile silicone and/or a non-silicone hydrophobic organic liquid, structured with an organic compound with amido groups, with optionally one or more polymeric or non-polymeric secondary structuring agents in small proportions. Among the secondary structuring agents, this document mentions polymers having siloxane groups and groups exhibiting hydrogen interactions without giving examples or results on a composition using these polymers.

It should be stated that the documents [7], [8] and [9] relate to deodorants for which the problems of non-stickiness and non-transfer of the composition do not exist as in the case of the make-up cosmetic products described above.

It is evident in the light of the preceding text that the transparency is, in all cases, an extremely delicate property to obtain for a cosmetic composition, in particular a solid cast composition of lipstick, for example in stick form, regardless of the nature of the silicone oils or the like constituting the fatty phase. Indeed, the transparency or the translucent character cannot be obtained if waxes are used for structuring the composition because, as indicated above, waxes have the disadvantage of making the composition opaque.

If it is sought to structure the composition, such as a stick, preferably without using waxes in order to avoid their opacifying effect, as is the case in the documents FR-A-2 817 740 [10] and FR-A-2 817 739 [11], other problems occur and the transparency is obtained at the detriment of other essential properties of the composition, such as the non-sticky character which is a property highly desired by the consumer.

It has not been possible until now to obtain a composition having, on the one hand, the attractive cosmetic appearance, which is represented by the transparency of the deposit and/or of the composition and, on the other hand, non-sticky and optionally transfer-free properties of the deposit formed from the said composition.

A need therefore exists for a cosmetic composition, in particular a solid cast composition, such as a lipstick, which is transparent or translucent and which furthermore gives a non-sticky deposit during its application. A need also exists for a cosmetic composition, in particular a coloured composition which is transparent or translucent and which makes it possible to obtain a deposit, which is in particular coloured, transparent, non-sticky, but also does not transfer upon contact.

### Disclosure of the invention

The aim of the invention is in particular to respond to the needs mentioned above.

In other words, the aim of the invention is to provide a care and/or make-up composition for the skin and/or the lips, which makes it possible to overcome the disadvantages and to solve the problems mentioned above, in particular the aim of the invention is to provide a composition which is transparent or translucent and which also makes it possible to obtain a deposit which is non-sticky and does not transfer through contact.

Surprisingly, the Applicant has found that the use of particular polymers, i.e. silicone polyamides, combined with a specific fatty phase comprising at least one ester-type oil, made it possible to achieve, inter alia, the above aims and to structure, in the absence of wax, the liquid fatty phases in the make-up or care compositions.

In particular, it has been observed that the compositions containing this combination of a particular polymer and a specific ester oil were not only transparent and translucent, but also resulted in non-sticky films or deposits with improved transfer-free properties.

The combination of these particular polymers with one or more ester oils present in the fatty phase makes it possible to obtain gels, in particular transparent or translucent, solid compositions having good mechanical strength and allowing a deposit in sufficient quantity, which is not sticky to the touch, which does not transfer upon contact and which is itself transparent or translucent.

The effects obtained by virtue of this combination of a particular polymer and a particular ester oil, in particular the combination of the non-transfer, of the absence of stickiness of the deposit and of the transparency or translucence of the composition and of the deposit, do not appear in the prior art documents.

The invention not only applies to make-up products for the lips such as lipsticks, lip pencils and lip glosses, but also to care products for the skin, including the scalp, and the lips, such as sun protection products in stick form for the skin, the face or the lips, or lip balms, to make-up products for the skin, both of the face and of the human body, such as foundations cast as a stick or in a dish, concealer products and temporary tattoo products, and to make-up products for the eyes such as eyeliners, in particular in pencil form, and mascaras, in particular cakes for keratinous fibres (eyelashes, eyebrows, hair).

More precisely, the subject of the invention is a care and/or make-up cosmetic composition, which is transparent or translucent and/or capable of giving a transparent or translucent deposit, comprising :
- a liquid fatty phase comprising at least one ester oil chosen from esters of monocarboxylic acids with monoalcohols, structured with at least one structuring polymer (homopolymer or copolymer) having a weight-average molecular mass ranging from 500 to 500 000, containing at least one moiety of formula (IV) or (V): 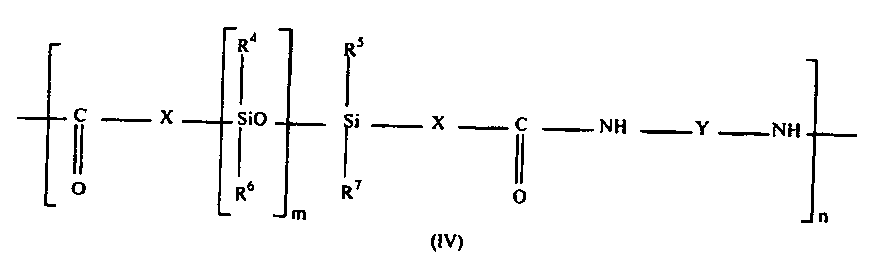 or 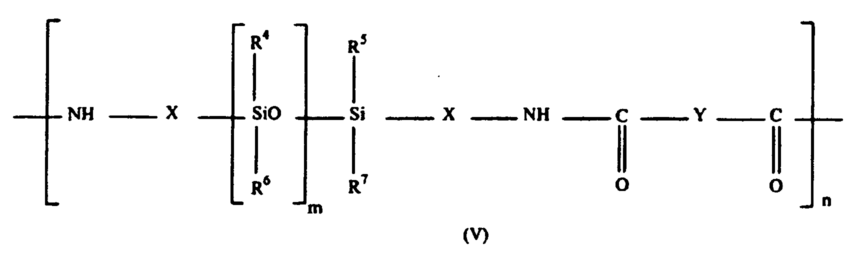 in which 1) R⁴, R⁵, R⁶ and R⁷, which may be identical or different, represent a group chosen from:
- linear, branched or cyclic, saturated or unsaturated, C₁ to C₄₀ hydrocarbon-based groups, possibly containing in their chain one or more oxygen, sulphur and/or nitrogen atoms, and possibly being partially or totally substituted with fluorine atoms,
- C₆ to C₁₀ aryl groups, optionally substituted with one or more C₁ to C₄ alkyl groups,
- polyorganosiloxane chains possibly containing one or more oxygen, sulphur and/or nitrogen atoms;
   2) the groups X, which may be identical or different, represent a linear or branched C₁ to C₃₀ alkylenediyl group, possibly containing in its chain one or more oxygen and/or nitrogen atoms;
   3) Y is a saturated or unsaturated, C₁ to C₅₀ linear or branched divalent alkylene, arylene, cycloalkylene, alkylarylene or arylalkylene group, possibly comprising one or more oxygen, sulphur and/or nitrogen atoms, and/or bearing as substituent one of the following atoms or groups of atoms: fluorine, hydroxyl, C₃ to C₈ cycloalkyl, C₁ to C₄₀ alkyl, C₅ to C₁₀ aryl, phenyl optionally substituted with 1 to 3 C₁ to C₃ alkyl groups, C₁ to C₃ hydroxyalkyl and C₁ to C₆ aminoalkyl, or
   4) Y represents a group corresponding to the formula: 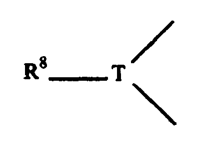 in which
- T represents a linear or branched, saturated or unsaturated, C₃ to C₂₄ trivalent or tetravalent hydrocarbon-based group optionally substituted with a polyorganosiloxane chain, and possibly containing one or more atoms chosen from O, N and S, or T represents a trivalent atom chosen from N, P and Al, and
- R⁸ represents a linear or branched C₁ to C₅₀ alkyl group or a polyorganosiloxane chain, possibly comprising one or more ester, amide, urethane, thiocarbamate, urea, thiourea and/or sulphonamide groups, which may possibly be linked to another chain of the polymer;
   5) the groups G, which may be identical or different, represent divalent groups chosen from: 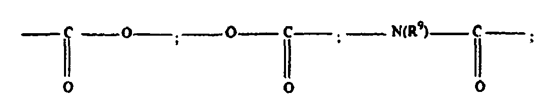 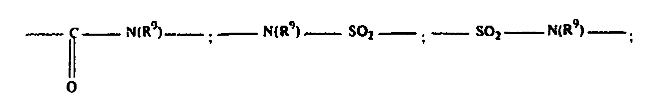 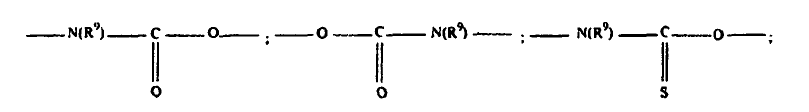 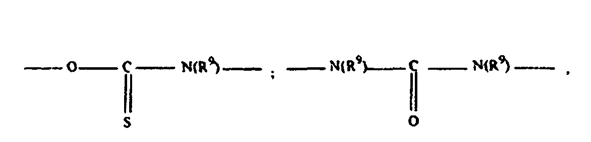 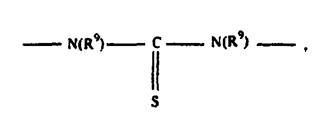 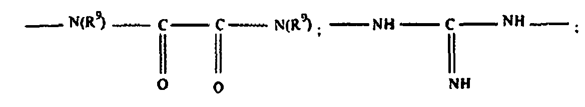 et 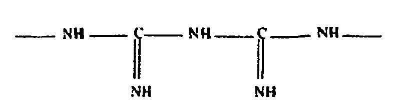 in which R⁹ represents a hydrogen atom or a linear or branched C₁ to C₂₀ alkyl group, on condition that at least 50% of the groups R⁹ of the polymer represent a hydrogen atom and that at least two of the groups G of the polymer are a group other than: 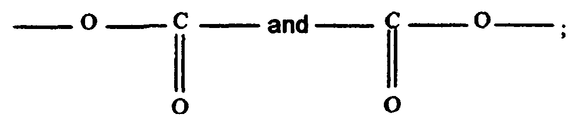
   6) n is an integer ranging from 2 to 500 and preferably from 2 to 200, and m is an integer ranging from 1 to 1 000, preferably from 1 to 700 and better still from 6 to 200,
- the polymer being solid at room temperature and soluble in the liquid fatty phase at a temperature of 25 to 250°C, and
the liquid fatty phase and the structuring polymer forming a physiologically acceptable medium.

Cosmetic compositions comprising a liquid fatty phase comprising a specific ester oil, which are transparent or translucent, and whose structuring is additionally provided by a specific polymer, for example of the silicone polyamide type (PASi), are not described in the prior art.

Surprisingly, it has been found that the use, in the fatty phase of the composition of the invention, of a specific oil which is a so-called "ester" oil, which can be called "short ester", made it possible to obtain a transparent or translucent composition.

In addition, the compositions according to the invention give transparent or translucent deposits or films.

In other words, the cosmetic compositions of the invention have as a characteristic, on the one hand, the transparency or the translucence of the deposit which is obtained therefrom and, on the other hand, the translucent or transparent appearance of the composition before its application, "in the pot", "in the mass".

This property of transparency or of translucence "in the mass" means that a layer of the composition having a given thickness, allows a portion of visible light to pass through. If this portion of visible light is scattered, the composition will be the mass, and if, on the contrary, it is not scattered, then the composition will be defined as being a composition which is transparent in the mass.

The transparent or translucent property of the composition according to the invention is determined in the following manner: the test composition is cast into a 30 ml Volga pot, the composition is allowed to cool (for 24 h at room temperature) and a white sheet is placed on top, on which a cross about 2 mm thick is drawn with a black felt pen. If the cross is visible to the naked eye in daylight at an observation distance of 40 cm, the composition is transparent or translucent.

This transparent or translucent appearance is very satisfactory, in particular for the consumer, from an aesthetic point of view and may therefore be of great commercial interest.

The transparent or translucent character of a cosmetic composition, for example in stick form, and what is more, of the deposit obtained therefrom, is extremely difficult to predict.

It is absolutely not possible to know in advance which particular component and in which proportion, will make it possible to obtain the transparency or the translucence of the composition and, a fortiori of the deposit.

The formulation of transparent or translucent cosmetic compositions is therefore totally unpredictable and can in no case be deduced from pre-existing formulations or the properties, for example the optical properties, of the compounds entering into these compositions.

Nothing could have suggested that by choosing the specific "short" esters among the considerable number of oils capable of being incorporated into the fatty phase of a cosmetic composition, a transparent or translucent composition and a deposit of this translucent or transparent composition would surprisingly be obtained, given the random and highly unpredictable character of the transparency and the translucent character of a composition and of a deposit thereof.

It is surprising that the combination of this specific ester with a specific structuring polymer, for example of the PASi type, leads to a composition which, while being transparent or translucent and giving a transparent or translucent deposit, also makes it possible to obtain a deposit which is "transfer-free" upon contact and which is not sticky.

Now, it has been found that only the specific esters according to the invention made it possible, among all the oils capable of being used in the cosmetic compositions, to obtain surprisingly, with the particular structuring polymers described above, and in the absence or in the presence of a small quantity of waxes, structured systems, such as gels or sticks exhibiting transparency/translucence, and formulations which are not sticky ("non-sticky") and which do not transfer upon contact.

The invention results from the double selection, from a very large number of oils and of structuring polymers respectively, of a specific oil and of a specific polymer whose combination surprisingly leads to a system which exhibits the combination of advantageous properties according to the invention.

In connection with the preceding text, the transfer-resistance of a cosmetic composition may be estimated by a transfer-resistance test which is well established in the technical field, such as the application of the made-up area on the skin or the lips (the so-called "kiss" test). The same applies to the non-sticky test (see Example 4).

The transparent or translucent composition of the invention may be provided in the form of a paste, a solid or a more or less viscous cream. It may be a simple or multiple, in particular an oil-in-water or a water-in-oil, a water-in-oil-in water or an oil-in-water-in oil, emulsion, or a rigid or soft gel having an oily continuous phase. The simple or multiple emulsion may comprise an aqueous or.oily continuous phase optionally containing dispersed lipid vesicles. In particular, it is provided in a form cast as a stick or in a dish and more especially in the form of an oily, in particular anhydrous, rigid gel and in particular of an anhydrous stick. More especially, it is provided in the form of a translucent or transparent rigid gel and preferably, if the composition contains pigments or dyes, a slightly coloured rigid gel, the liquid fatty phase forming the continuous phase. An anhydrous composition will comprise less than 10% by weight of water, for example less than 5% by weight.

The structuring of the liquid fatty phase can be modulated according to the nature of the structuring polymer and the ester oil used; this structuring may be such that a rigid structure is obtained in the form of a baton or a stick, having good mechanical strength, which is transparent or translucent. When they are coloured, these batons make it possible, after application, to obtain a deposit which is transparent, translucent, non-transfer and non-sticky.

The composition may contain one or more structuring polymers, as defined above, and one or more ester oils.

Advantageously, the composition of the invention is a composition for the lips and even better a lipstick composition in particular in stick form.

### Liquid fatty phase

The liquid fatty phase of the composition of the invention comprises one or more oils.

According to the invention, at least one of these oils is an oil called "ester "oil" which is chosen from esters of monocarboxylic acids with monoalcohols.

Advantageously, the said ester corresponds to the following formula (I):

R₁-CO-O-R₂ (I)

where R₁ represents a linear or branched alkyl radical of 1 to 40 carbon atoms, preferably of 7 to 19 carbon atoms, optionally comprising one or more ethylenic double bonds, and optionally substituted,

R₂ represents a linear or branched alkyl radical of 1 to 40 carbon atoms, preferably of 3 to 30 carbon atoms and even better of 3 to 20 carbon atoms, optionally comprising one or more ethylenic double bonds, and optionally substituted.

The expression "optionally substituted" is understood to mean that R₁ and/or R₂ may carry one or more substituents chosen for example from groups comprising one or more heteroatoms chosen from O, N and S, such as amino, amine, alkoxy, hydroxyl.

Preferably, the total number of carbon atoms of R₁ + R₂ is ≥ 9.

The ester used according to the invention may be designated under the name of "short" ester.

R₁ may represent the residue of a preferably higher, linear or, preferably branched fatty.acid comprising from 1 to 40 and even better from 7 to 19 carbon atoms and R₂ may represent a linear or preferably branched hydrocarbon chain containing from 1 to 40, preferably from 3 to 30 and even better from 3 to 20 (19 to 28, 8 to 27, 7 to 26 C) carbon atoms. Again, preferably the number of carbon atoms of R₁ + R₂ ≥ 9.

Examples of R₁ groups are those derived from fatty acids chosen from the group consisting of acetic, propionic, butyric, caproic, caprylic, pelargonic, capric, undecanoic, lauric, myristic, palmitic, stearic, isostearic, arachidic, behenic, oleic, linolenic, linoleic, oleostearic, arachidonic and erucic acids, and mixtures thereof.

Examples of esters which can be used in the fatty phases of the compositions of the invention are for example purcellin oil (cetostearyl octanoate), isononyl isononanoate, isopropyl myristate, 2-ethylhexyl palmitate, 2-octyldodecyl stearate, 2-octyldodecyl erucate, isostearyl isostearate, and heptanoates, octanoates, decanoates or ricinoleates of alcohols, for example of fatty alcohols.

Advantageously, the esters are chosen from the compounds of formula (I) above, in which R₁ represents an unsubstituted linear or branched alkyl group, optionally comprising one or more ethylenic double bonds, from 1. to 40 carbon atoms, preferably from 7 to 19 carbon atoms; and R₂ represents an unsubstituted linear or branched alkyl group, optionally comprising one or more ethylenic double bonds, from 1 to 40 carbon atoms, preferably from 3 to 30 carbon atoms, and even better from 3 to 20 carbon atoms.

Preferably, R₁ is an unsubstituted branched alkyl group of 4 to 14 carbon atoms, preferably of 8 to 10 carbon atoms, and R₂ is an unsubstituted branched alkyl group of 5 to 15 carbon atoms, preferably of 9 to 11 carbon atoms. Preferably, in formula (I); R₁-CO- and R₂ have the same number of carbon atoms and are derived from the same radical, preferably an unsubstituted branched alkyl, for example isononyl, that is to say that advantageously the ester oil molecule is symmetrical.

The ester oil will be preferably chosen from the following compounds:
- isononyl isononanoate,
- cetostearyl octanoate,
- isopropyl myristate,
- 2-ethylhexyl palmitate,
- 2-octyldodecyl stearate,
- 2-octyldodecyl erucate,
- isostearyl isostearate.

The ester which is preferred amongst all is isononyl isononanoate which makes it possible to obtain at best compositions having excellent transparency or translucence combined with excellent properties of non-transfer and absence of stickiness.

Advantageously, the fatty phase comprises from 0.5 to 100% by weight of oil or of ester oils, preferably from 1 to 80% by weight, still more preferably from 2 to 50% by..weight, even better from 2 to 40% by weight.

In these percentage ranges, the surprising effects of the invention, namely essentially the combination of transparency/translucence, non-transfer, and non-stickiness are exhibited with the greatest magnitude.

Preferably, the fatty phase comprises 100% by weight of the ester oil(s), that is to say that the fatty phase contains, as oils, only ester oils as described above, excluding any other oil.

More preferably still, the fatty phase comprises only one ester oil (and not a mixture) which is preferably isononyl isononanoate, this single ester oil being present in the above percentages, and preferably in an amount of 100% by weight.

The liquid fatty phase of the composition according to the invention comprises at least one oil generally chosen from hydrocarbon, silicone and fluorinated oils.

An oil is a nonaqueous compound which is immiscible with water.

The liquid fatty phase may comprise at least one volatile oil.

For the purposes of the invention, a volatile oil has a flash point preferably of 35 to 135°C (measured according to the method) or no flash point. The flash point is the temperature at which the vapours emitted by a fuel ignite upon contact with a flame, a spark or a heat source.

Volatile oils have advantageously at room temperature (25°C) and atmospheric pressure (760 mmHg) a vapour pressure ranging from 0.01 mm to 300 mmHg (1.33 Pa to 40 000 Pa) and even better ranging from 0.05 to 190 mmHg (6.65 Pa to 25 330 Pa). According to the invention, the volatile oil may be chosen from linear, branched or cyclic silicone oils having a flash point equal to or greater than 40°C and/or a viscosity of less than 8 cSt, such as linear, branched or cyclic polydimethylsiloxanes (PDMS) having from 3 to 7 silicon atoms.

By way of examples of such volatile oils, there may be mentioned the compounds given in Table 1 below.

The composition may contain a non-volatile silicone oil.

The silicone oils of the invention have a viscosity which is advantageously chosen from the ranging going from 5 to 800 000 cst at 25°C, preferably from 10 to 500 000 cSt, and even better from 10 to 5 000 cSt.

The non-volatile silicone oils may be polydimethylsiloxanes, polyalkylmethylsiloxanes, dimethicone copolyols, alkylmethicone copolyols, cetyldimethicone, silicones with alkylglyceryl ether groups, silicones with side amine groups and dilauroyltrimethylol propane siloxysilicate. The alkyl groups of these oils have in particular from 2 to 24 carbon atoms.

The non-volatile silicone oils may be polydimethylsiloxanes, polyalkylmethylsiloxanes, dimethicone copolyols, alkylmethicone copolyols, cetyldimethicone, silicones with alkylglyceryl ether groups, silicones with side amine groups and dilauroyltrimethylol propane siloxysilicate. The alkyl groups of these oils have in particular from 2 to 24 carbon atoms.

The non-volatile silicone oils which can be used in the invention may be in particular linear, non-volatile polydimethylsiloxanes (PDMS) which are liquid at room temperature; polydimethylsiloxanes containing alkyl, alkoxy or phenyl groups, which are pendent and/or at the silicone chain end, groups each having from 2 to 24 carbon atoms; phenylated silicones such as phenyltrimethicones, phenyldimethicones, phenyltrimethylsiloxy diphenylsiloxanes, diphenyl-dimethicones, diphenylmethyldiphenyltrisiloxanes, 2-phenylethyl trimethylsiloxysilicates, fluorinated silicones with one or more group(s) that is (are) pendent or at the chain end having from 1 to 12 carbon atoms of which all or some of the hydrogen atoms are substituted with fluorine atoms, dimethiconols and mixtures thereof.

**Table 1**

| **Compound** | **Flash point (°C)** | **Viscosity (cSt)** |
|---|---|---|
| Octyltrimethicone | 93 | 1.2 |
| Hexyltrimethicone | 79 | 1.2 |
| Decamethylcyclopentasiloxane (cyclopentasiloxane or D5) | 72 | 4.2 |
| Octamethylcyclotetrasiloxane (cyclotetradimethylsiloxane or D4) | 55 | 2.5 |
| Dodecamethylcyclohexasiloxane (D6) | 93 | 7 |
| Decamethyltetrasiloxane (L4) | 63 | 1.7 |
| KF 96 A from Shin Etsu | 94 | 6 |
| PDMS (polydimethylsiloxane) DC 200 (1.5 cSt) from Dow Corning | 56 | 1.5 |
| PDMS DC 200 (2 cSt) from Dow Corning | 87 | 2 |
| PDMS DC 200 (5 cSt) | 134 | 5 |
| from Dow Corning | | |
| PDMS DC 200 (3 cSt) from Dow Corning | 102 | 3 |

In other words, the volatile silicone oil(s) may be chosen, for example, from the group consisting of the compounds -of Table 1, heptamethyloctyltrisiloxane, dodecamethylpentasiloxane and mixtures thereof.

The volatile-silicone oil-may also be chosen from the group comprising fluorinated.silicone oils such as silicones with alkyl and perfluoroalkyl groups, silicones with oxyethylenated/oxypropylenated (EO/PP) side groups and with perfluorinated groups, silicones with perfluorinated side groups and with glycerolated side groups, perfluoroalkylmethylphenylsiloxanes, these oils having a vapour pressure greater than or equal to 0.02 mmHg.

The volatile non-silicone oils may be chosen from the group comprising hydrocarbon oils and volatile esters and ethers such as volatile hydrocarbons such as isododecane and isohexadecane, C₈-C₁₆ isoparaffins, isohexyl or isodecyl neopentanoates.

The volatile oil may also be chosen from fluorinated oils such as perfluoropolyethers, perfluoroalkanes such as perfluorodecalin, perfluorodamantanes, esters (monoesters, diesters and triesters) of perfluoroalkyl phosphates and fluorinated ester oils.

By way of example of volatile non-silicone oils which can be used in the invention, there may be mentioned the compounds of Table 2 which follows.

**Table 2**

| **Compound** | **Flash point (°C)** |
|---|---|
| Isododecane | 43 |
| Isohexadecane | 102 |
| Isodecyl neopentanoate | 118 |
| Propylene glycol n-butyl ether | 60 |
| Ethyl 3-ethoxypropionate | 58 |
| Propylene glycol methyl ether | 46 |
| acetate* | |
| Isopar L (C₁₁-C₁₃ isoparaffin) | 62 |
| Isopar H (C₁₁-C₁₂ isoparaffin) | 56 |

The liquid fatty phase advantageously contains at least 1%, and even better at least 5%, for example from 10 to 90%, by weight of silicone oil(s) advantageously having a viscosity of less than 1 000 cSt and even better of less than 100 cSt because the silicone polymers used in the invention are more soluble in silicone oils of low viscosity.

When the fatty phase comprises a volatile oil, it advantageously represents from 3 to 89.4%, and even better from 5 to 60%, for example from 5 to 20% of the total weight of the composition.

The liquid fatty phase may also contain other non-silicone oils, for example polar oils such as:
- hydrocarbonaceous vegetable oils with a high content of triglycerides consisting of esters of fatty acids and of glycerol in which the fatty acids may have varied chain lengths, it being possible for the latter to be linear or branched, saturated or unsaturated; these oils are in particular wheat germ, maize, sunflower, karite, castor, sweet almond, macadamia, apricot, soybean, rapeseed, cottonseed, lucerne, poppy seed, pumpkin seed, sesame, gourd, avocado, hazelnut, grapeseed or blackcurrant seed, evening primrose, millet, barley, quinoa, olive, rye, safflower, candlenut, passion flower and rose musk oils; or triglycerides of caprylic/capric acids such as those sold by the company Stearines Dubois or those sold under the names Miglyol 810, 812 and 818 by the company Dynamit Nobel;
- synthetic esters having from 10 to 40 carbon atoms;
- C₈ to C₂₆ fatty alcohols such as oleyl alcohol and octyldodecanol;
- fatty acids such as oleic, linoleic or linolenic acid; and
- mixtures thereof.

The liquid fatty phase may also contain apolar oils such as linear or branched hydrocarbons or fluorocarbons of synthetic or mineral origin, which are volatile or not, such as volatile oils of paraffin (such as isoparaffins, isododecane) or non-volatile oils of paraffin and its derivatives, petroleum jelly, polydecenes, hydrogenated polyisobutene such as parleam, squalane, and mixtures thereof.

Thus, the invention may be used for example with the following different fatty phases:
1) a fatty phase consisting solely of a mixture of ester oils;
2) a fatty phase consisting solely of a single ester oil;
3) a fatty phase consisting of a mixture of oils comprising at least one ester oil and at least one volatile silicone oil;
4) a fatty consisting of a mixture of oils comprising at least one ester oil and at least one non-silicone volatile oil;
5) a fatty phase consisting a mixture of oils comprising at least one ester oil, at least one non-silicone volatile oil and optionally one volatile silicone oil;
6) a fatty phase consisting of a mixture of oils comprising at least one ester oil, one non-volatile, non-silicone oil and optionally at least one volatile non-silicone oil; and
in cases 3), 4) and 5), the mixture may also comprise a non-volatile non-silicone oil.

Generally, the liquid fatty phase represents from 5 to 95% of the total weight of the composition and even better from 20 to 75%.

According to the invention, the composition comprises, in addition, solid particles, chosen from fillers and pigments (including pearlescent pigments), and mixtures thereof, in a quantity such that the composition remains transparent or translucent.

The expression pigments is understood to mean any solid particle insoluble in the composition which serves to give and/or modify a colour and/or an iridescent appearance.

The pigments may be white or coloured, inorganic and/or organic, coated or not. Inorganic pigments may be chosen, for example, from zinc oxides, iron oxides, titanium oxides and mixtures thereof. There thus may be mentioned, among the inorganic pigments, titanium or zinc dioxide, optionally surface-treated, zirconium or cerium oxides, and iron or chromium oxides, manganese violet, ultramarine blue, chromium hydrate and ferric blue. Among the organic pigments, there may be mentioned carbon black, pigments of the D & C type, and lacquers based on carmine, barium, strontium, calcium or aluminium. The pigments may represent from 0.1 to 30% of the total weight of the composition, preferably from 2 to 15%, if they are present.

### Structuring silicone polymer

The structuring polymer(s) of the composition are solid at room temperature (25°C) and atmospheric pressure (760 mmHg) and are soluble in the liquid fatty phase at a temperature of 25 to 250°C.

The expression polymer is understood to mean, for the purpose of the invention, a compound having at least 2 repeating moieties, preferably at least 3. repeating moieties and even better 10 repeating moieties.

In the composition of the invention, the structuring silicone polymer-of the gelling system generally represents from 0.5 to 80%, preferably from 2 to 60% and even better from 5 to 40% of the total weight of the composition.

Moreover, the structuring polymer/total oil(s) mass ratio of the composition is preferably from 0.01 to 0.8, more preferably from 0.05 to 0.5 and better still from 0.1 to 0.5.

The structuring polymer/ester oil mass ratio is generally from 0.01 to 10, preferably from 0.05 to 5 and more preferably from 0.1 to 2.

The polymers used as structuring agents in the composition of the invention are polymers of the polyorganosiloxane type such as those described in the documents US-A-5 874 069 [4], US-A-5, 919, 441 [5], US-A-6, 051, 216 [6] and US-A-5, 981, 680 [12].

According to the invention, the groups capable of establishing hydrogen interactions are amide groups of formulae -C(O)NH- and -HN-C(O)-.

In this case, the structuring agent is a polymer comprising at least one moiety of formula (IV) or (V): 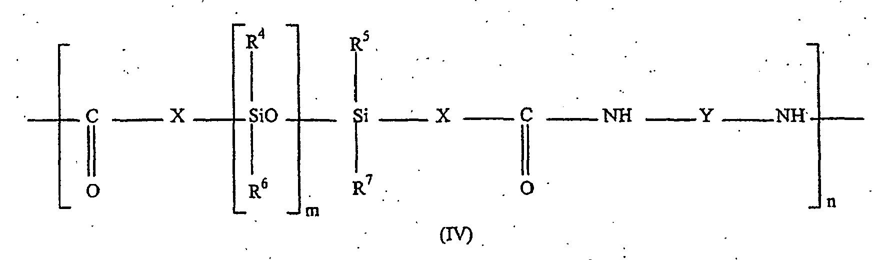 or 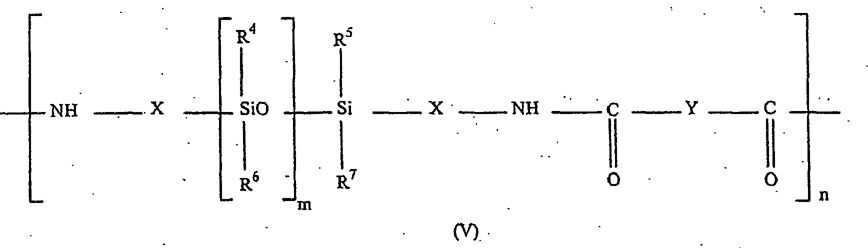 in which R⁴, R⁵, R⁶, R⁷, X, Y, m and n are as defined above.

Such a moiety may be obtained:
- either by a condensation reaction between a silicone containing α,ω-carboxylic acid ends and one or more diamines, according to the following reaction scheme: 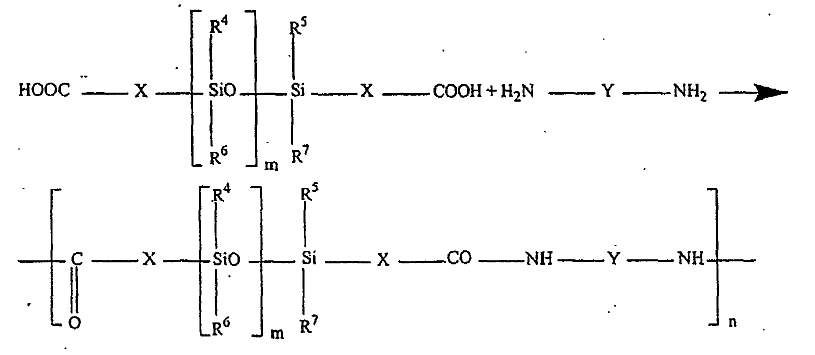
- or by reaction of two molecules of α-unsaturated carboxylic acid with a diamine according to the following reaction scheme:

   CH₂=CH-X¹-COOH+H₂N-Y-NH₂ → CH₂=CH-X¹-CO-NH-Y-NH-CO-X¹-CH=CH₂

   followed by the addition of a siloxane to the ethylenic unsaturations, according to the following scheme:

   CH₂=CH-X¹-CO-NH-Y-NH-CO-X¹-CH=CH₂

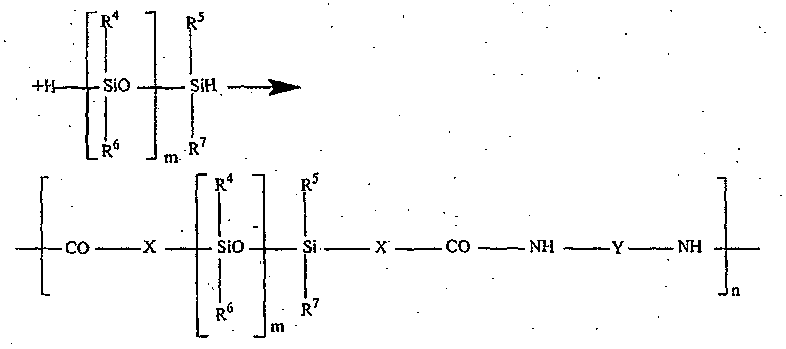

   in which X¹-(CH₂)₂- corresponds to X defined above and Y, R⁴, R⁵, R⁶, R⁷ and m are as defined above;
- or by reaction of a silicone containing α,ω-NH₂ ends and a diacid of formula HOOC-Y-COOH according to the following reaction scheme:

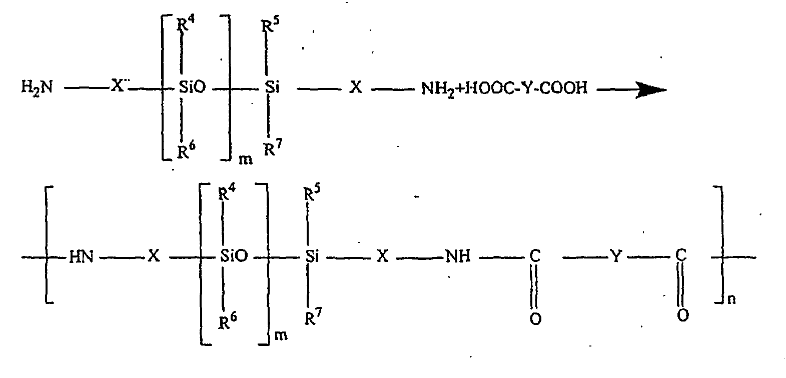

In these polyamides of formula (IV) or (V), m is preferably in the range from 1 to 700, more preferably from 15 to 500 and better still from 10 to 100, and n is in particular in the range from 1 to 500, preferably from 1 to 100 and better still from 4 to 25, X is preferably a linear or branched alkylene chain containing from 1 to 30 carbon atoms and in particular 3 to 10 carbon atoms, and
- Y is preferably an alkylene chain that is linear or branched or that possibly comprises rings and/or unsaturations, containing from 1 to 40 carbon atoms, in particular from 1 to 20 carbon atoms and better still from 2 to 6 carbon atoms, in particular 6 carbon atoms.

In formulae (IV) and (V), the alkylene group representing X or Y can optionally contain in its alkylene portion at least one of the following elements:
1) 1 to 5 amide, urea, urethane or carbamate groups,
2) a C₅ or C₆ cycloalkyl group, and
3) a phenylene group optionally substituted with 1 to 3 identical or different C₁ to C₃ alkyl groups.

In formulae (IV) and (V), the alkylene groups may also be substituted with at least one element chosen from the group consisting of:
- a hydroxyl group,
- a C₃ to C₈ cycloalkyl group,
- one to three C₁ to C₄₀ alkyl groups,
- a phenyl group optionally substituted with one to three C₁ to C₃ alkyl groups,
- a C₁ to C₃ hydroxyalkyl group, and
- a C₁ to C₆ aminoalkyl group.

In these formulae (IV) and (V), Y may also represent: 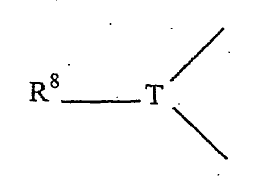 in which R⁸ represents a polyorganosiloxane chain and T represents a group of formula: 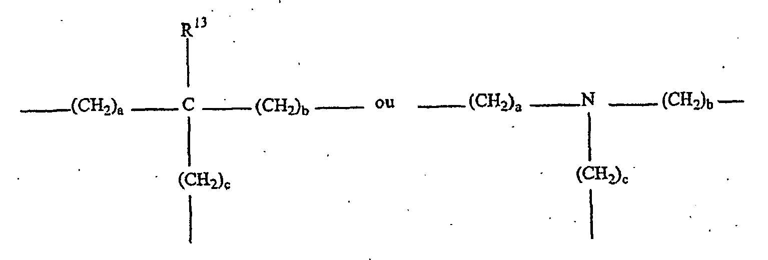 in which a, b and c are, independently, integers ranging from 1 to 10, and R¹³ is a hydrogen atom or a group such as those defined for R⁴, R⁵, R⁶ and R⁷.

In formulae (IV) and (V), R⁴, R⁵, R⁶ and R⁷ preferably represent, independently, a linear or branched C₁ to C₄₀ alkyl group, preferably a CH₃, C₂H₅, n-C₃H₇ or isopropyl group, a polyorganosiloxane chain or a phenyl group optionally substituted with one to three methyl or ethyl groups.

As has been seen previously, the polymer may comprise identical or different moieties of formula (IV) or (V).

Thus, the polymer may be a polyamide containing several moieties of formula (IV) or (V) of different lengths, i.e. a polyamide corresponding to the formula: 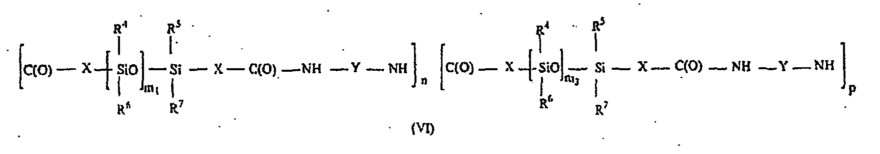 in which X, Y, n and R⁴ to R⁷ have the meanings given above, m₁ and m₂, which are different, are chosen in the range from 1 to 1 000, and p is an integer ranging from 2 to 300.

In this formula, the moieties may be structured to form either a block copolymer, or a random copolymer or an alternating copolymer. In this copolymer, the moieties may be not only of different lengths, but also of different chemical structures, for example containing different groups Y. In this case, the copolymer may correspond to the formula: 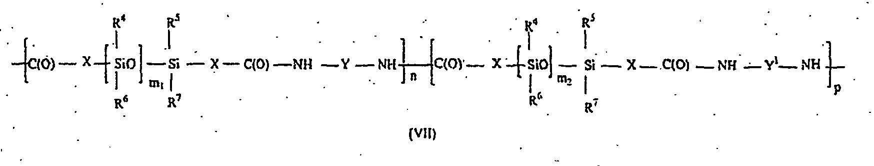 in which R⁴ to R⁷, X, Y, m₁, m₂, n and p have the meanings given above and Y¹ is different from Y but chosen from the groups defined for Y. As previously, the various moieties may be structured to form either a block copolymer, or a random copolymer or an alternating copolymer.

In this first embodiment of the invention, the structuring agent may also consist of a graft copolymer. Thus, the polyamide containing silicone units may be grafted and optionally crosslinked with silicone chains containing amide groups. Such polymers may be synthesized with trifunctional amines.

In this case, the copolymer may comprise at least one moiety of formula: 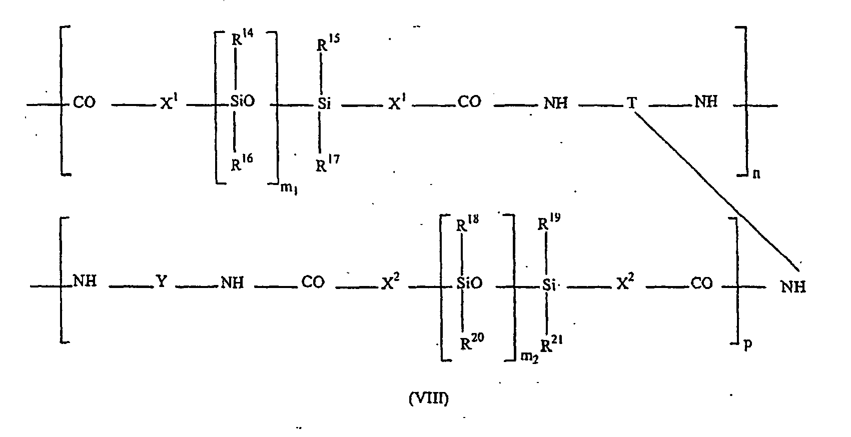 in which X¹ and X², which are identical or different, have the meaning given for X in formula (II), n is as defined in formula (II), Y and T are as defined in formula (II), R¹⁴ to R²¹ are groups chosen from the same group as R⁴ to R⁷, m₁ and m₂ are numbers in the range from 1 to 1 000, and p is an integer ranging from 2 to 500.

In formula (VIII), it is preferred that:
- p is in the range from 1 to 25 and better still from 1 to 7,
- R¹⁴ to R²¹ are methyl groups,
- T corresponds to one of the following formulae: 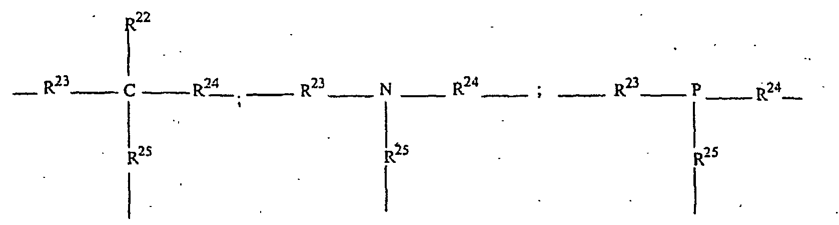 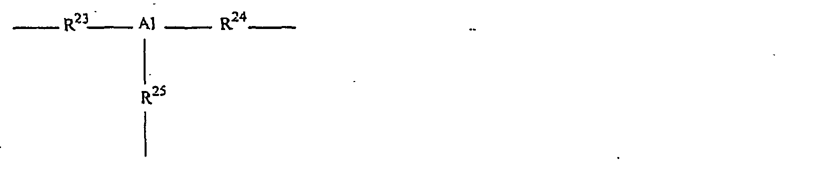
in which R²² is a hydrogen atom or a group chosen from the groups defined for R⁴ to R⁷, and R²³, R²⁴ and R²⁵ are, independently, linear or branched alkylene groups, and more preferably corresponds to the formula: 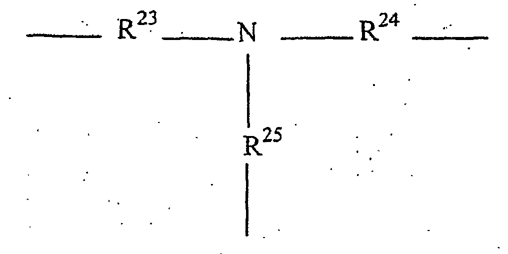 in particular with R²³, R²⁴, and R²⁵ representing -CH₂-CH₂-,
- m₁ and m₂ are in the range from 15 to 500 and better still from 15 to 45,
- X¹ and X² represent -(CH₂)₁₀-, and
- Y represents -CH₂-.

These polyamides containing a grafted silicone moiety of formula (VIII) may be copolymerized with polyamide-silicones of formula (III) to form block copolymers, alternating copolymers or random copolymers. The weight percentage of grafted silicone moieties (VIII) in the copolymer may range from 0.5% to 30% by weight.

According to the invention, as has been seen previously, the siloxane units may be in the main chain or backbone of the polymer, but they may also be present in grafted or pendent chains. In the main chain, the siloxane units may be in the form of segments as described above. In the pendent or grafted chains, the siloxane units may appear individually or in segments.

According to the invention, the preferred siloxane-based polyamides are:
- polyamides of formula (IV) in which m is from 15 to 50;
- polyamides of formula (IV) in which m is from 30 to 500;
- mixtures of two or more polyamides in which at least one polyamide has a value of m in the range from 15 to 50 and at least one polyamide has a value of m in the range from 30 to 50;
- polymers of formula (VI) with m₁ chosen in the range from 15 to 50 and m₂ chosen in the range from 30 to 500 with the portion corresponding to m₁ representing 1% to 99% by weight of the total weight of the polyamide and the portion corresponding to m₂ representing 1% to 99% by weight of the total weight of the polyamide;
- mixtures of polyamide of formula (IV) combining
   1) 80% to 99% by weight of a polyamide in which n is equal to 2 to 10 and in particular 3 to 6, and
   2) 1% to 20% of a polyamide in which n is in the range from.30 to 500 and in particular from 30 to 100,
- mixtures of polyamide of formula (IV) combining
   1) 1% to 20% by weight of a polyamide in which n is equal to 2 to 10 and in particular 3 to 6, and
   2) 80% to 99% of a polyamide in which n is in the range from 30 to 500 and in particular from 30 to 100;
- polyamides corresponding to formula (VII) in which at least one of the groups Y and Y¹ contains at least one hydroxyl substituent;
- polyamides of formula (IV) synthesized with at least one portion of an activated diacid (diacid chloride, dianhydride or diester) instead of the diacid;
- polyamides of formula (IV) in which X represents
- (CH₂) ₃- or - (CH₂)₁₀; and
- polyamides of formula (IV) in which the polyamides end with a monofunctional chain chosen from the group consisting of monofunctional amines, monofunctional acids, monofunctional alcohols, including fatty acids, fatty alcohols and fatty amines, such as, for example, octylamine, octanol, stearic acid and stearyl alcohol.

According to the invention, the ends of the polymer chains may end with:
- a C₁ to C₅₀ alkyl ester group by introducing a C₁ to C₅₀ monoalcohol during the synthesis,
- a C₁ to C₅₀ alkylamide group by taking as stopping group a monoacid if the silicone is α,ω-diaminated, or a monoamine if the silicone is an α,ω-dicarboxylic acid.

According to one embodiment variant of the invention, it is possible to use a copolymer of silicone polyamide and of hydrocarbon-based polyamide, i.e. a copolymer comprising moieties of formula (IV) or
(V) and hydrocarbon-based polyamide moieties. In this case, the polyamide-silicone moieties may be arranged at the ends of the hydrocarbon-based polyamide.

Polyamide-based structuring agents containing silicones may be produced by silylic amidation of polyamides based on fatty acid dimer. This approach involves the reaction of free acid sites existing on a polyamide as end sites, with oligosiloxane-monoamines and/or oligosiloxane-diamines (amidation reaction), or alternatively with oligosiloxane alcohols or oligosiloxane diols (esterification reaction). The esterification reaction requires the presence of acid catalysts, as is known in the art. It is desirable for the polyamide containing free acid sites, used for the amidation or esterification reaction, to have a .. relatively high number of acid end groups (for example polyamides with high acid numbers, for example from 15 to 20).

For the amidation of the free acid sites of the hydrocarbon-based polyamides, siloxane diamines with 1 to 300, more particularly 2 to 50 and better still 2, 6, 9.5, 12, 13.5, 23 or 31 siloxane groups, may be used for the reaction with hydrocarbon-based polyamides based on fatty acid dimers. Siloxane diamines containing 13.5 siloxane groups are preferred, and the best results are obtained with the siloxane diamine containing 13.5 siloxane groups and polyamides containing high numbers of carboxylic acid end groups.

The reactions may be carried out in xylene to extract the water produced from the solution by azeotropic distillation, or at higher temperatures (about 180 to 200°C) without solvent. Typically, the efficacy of the amidation and the reaction rates decrease when the siloxane diamine is longer, that is to say when the number of siloxane groups is higher. Free amine sites may be blocked after the initial amidation reaction of the diaminosiloxanes by reacting them either with a siloxane acid, or with an organic acid such as benzoic acid.

For the esterification of the free acid sites on the polyamides, this may be performed in boiling xylene with about 1% by weight, relative to the total weight of the reagents, of para-toluenesulphonic acid as catalyst.

These reactions carried out on the carboxylic acid end groups of the polyamide lead to the incorporation of silicone moieties only at the ends of the polymer chain.

It is also possible to prepare a copolymer of polyamide-silicone, using a polyamide containing free amine groups, by amidation reaction with a siloxane containing an acid group.

It is also possible to prepare a structuring agent based on a copolymer between a hydrocarbon-based polyamide and a silicone polyamide, by transamidation of a polyamide having, for example, an ethylenediamine constituent, with an oligosiloxane-α,ω-diamine, at high temperature (for example 200 to 300°C), to carry out a transamidation such that the ethylenediamine component of the original polyamide is replaced with the oligosiloxane diamine.

The copolymer of hydrocarbon-based polyamide and of polyamide-silicone may also be a graft copolymer comprising a hydrocarbon-based polyamide backbone with pendent oligosiloxane groups.

This may be obtained, for example:
- by hydrosilylation of unsaturated bonds in polyamides based on fatty acid dimers;
- by silylation of the amide groups of a polyamide; or
- by silylation of unsaturated polyamides by means of an oxidation, that is to say by oxidizing the unsaturated groups into alcohols or diols, to form hydroxyl groups that are reacted with siloxane carboxylic acids or siloxane alcohols. The olefinic sites of the unsaturated polyamides may also be epoxidized and the epoxy groups may then be reacted with siloxane amines or siloxane alcohols.

As examples of polymers that are used, mention may be made of the silicone polyamides obtained in accordance with Examples 1 to 3 of document US-A-5 981 680.

The structuring polymers and copolymers used in the composition of the invention advantageously have a softening point from 65°C to 190°C. Preferably, they have a softening point ranging from 70 to 130°C and better still from 80°C to 105°C. This softening point is lower than that of the other known structuring polymers, which facilitates the use of the polymers described above, allows the use of volatile oils and limits the deteriorations of the liquid fatty phase.

They have good solubility in silicone oils and ester oils and produce macroscopically homogeneous compositions. Preferably, they have an average molecular mass from 500 to 200 000, for example from 1 000 to 100 000, preferably from 2 000 to 30 000.

The quantities of the ester ("short" ester) oil(s) and of the structuring polymer (s) may be chosen according to the desired texture or hardness and the desired stability of the compositions and according to the specific application envisaged. The respective quantities of the (at least one) structuring polymer and of the short ester(s) may be such that a disintegratable solid which does not run under the effects of its own weight is obtained.

According to the invention, the composition preferably has a hardness ranging from 20 to 2 000 gf and better still from 20 to 900 gf, particularly from 20 to 600 gf, and for example from 150 to 450 gf. This hardness may be measured according to a method of penetration of a probe into the said composition and in particular with the aid of a texture analyser (for example TA-TXT2i from Rheo) equipped with an ebonite cylinder 25 mm in height and 8 mm in diameter. The hardness measurement is carried out at 20°C at the centre of five samples of the said composition. The cylinder is introduced into each sample of composition at a pre-speed of 2 mm/s, then at a speed of 0.5 mm/s and finally at a post-speed of 2 mm/s, the total displacement being 1 mm. The recorded hardness value is that of the maximum peak. The measurement error is ± 50 gf.

The hardness may also be measured by the "cheese wire" method, which consists in cutting a tube of lipstick 12.7 mm or 8.1 mm in diameter and in measuring the hardness at 20°C, using a DFGHS 2 tensile testing machine from the company Indelco-Chatillon, travelling at a speed of 100 mm/minute. It is expressed as the shear force (expressed in gram-force) required to cut a stick under these conditions. According to this method, the hardness of a composition in stick form according to the invention ranges from 30 to 300 gf, preferably from 30 to 250 gf and for example from 30 to 200 gf, even better from 30 to 180 gf, when the diameter of the stick is equal to 12.7 mm.

The hardness of the composition according to the invention is such that the composition is self-supporting and can disintegrate easily to form a satisfactory deposit on the skin and the lips. In addition, with this hardness, the composition of the invention shows good impact strength.

According to the invention, the composition in stick form has the behaviour of a deformable and supple elastic solid, giving noteworthy elastic softness on application.

The respective amounts of ester oil and of silicone polymer are chosen according to the desired gel hardness and depending on the particular application envisaged. The respective quantities of polymer and of ester oil should be such that they allow the production of a self-supported composition, for example in the form of a disintegrable stick. In practice, the quantity of polymer (as active material) represents from 0.5 to 80% of the total weight of the composition, and even better from 5 to 40%.

Generally, the silicone polymer/ester oil mass ratio is in the range from 0.01 to 0.8, preferably from 0.05 to 0.5 and preferably still from 0.1 to 0.5.

The structuring silicone polymer preferably represents from 5 to 30% by weight of the composition, preferably still from 0.5 to 30% by weight, even better from 1 to 30% by weight.

### Other additives

The composition of the invention may also comprise any ingredient usually used in the field under consideration.

Needless to say, the person skilled in the art will take care to select the optional additional ingredients and/or the amount thereof such that the advantageous properties of the composition according to the invention are not, or are not substantially, adversely affected by the envisaged addition.

The composition according to the invention may be in the form of an optionally tinted, transparent or translucent, dermatological or care composition for keratinous materials such as the skin, the lips and/or the superficial body growths, in the form of a sun protection composition, or of a make-up-removing product, in stick form or in cast form. It can especially be used as a care base for the skin, the superficial body growths or the lips (lip balms, for protecting the lips against the cold and/or sunlight and/or the wind, or a care cream for the skin, the nails or the hair).

The composition of the invention may be provided in particular in the form of a transparent or translucent rigid gel, in particular in.the form of a transparent anhydrous stick.

The composition of the invention may also be in the form of a coloured, transparent or translucent, make-up product for the skin, in particular a foundation, optionally having care or treatment properties, a blusher, a face powder, an eyeshadow, a concealer product, an eyeliner or a make-up product for the body; a lip make-up, for instance a lipstick, a lip gloss or a pencil, optionally having care or treatment properties; a make-up for the superficial body growths, for instance the nails or the eyelashes, in particular in the form of a mascara cake, or for the eyebrows and the hair, especially in the form of a pencil.

Needless to say, the composition of the invention must be cosmetically or dermatologically acceptable, that is to say that it must contain a non-toxic physiologically acceptable medium that can be applied to the skin, the superficial body growths or the lips of human beings. For the purposes of the invention, the expression "cosmetically acceptable" means a composition of pleasant appearance, odour and feel.

According to the invention, the composition may furthermore contain colouring matter which may be chosen from lipophilic dyes, hydrophilic dyes, and mixtures thereof.

The composition according to the invention may be manufactured by known methods, generally used in the cosmetic or dermatological field. It may be manufactured by the method which consists in heating the polymer at least to its softening point, adding thereto the oil(s), and then in mixing the whole until a clear solution is obtained. The colouring matter and/or the solid particles, and the additives are then added, with stirring. The homogeneous mixture obtained can then be cast in a suitable mould such as a lipstick mould, or directly into the packaging articles (especially a case or dish).

The subject of the invention is also a make-up structured solid composition for the skin, the lips and/or the superficial body growths, containing at least one pigment or dye in a sufficient quantity for applying make-up to the skin, the lips and/or the superficial body growths and a liquid continuous fatty phase comprising at least one ester oil chosen from esters of monocarboxylic acids with monoalcohols and polyalcohols, structured with at least one polymer (homopolymer or copolymer) having a weight-average molecular mass ranging from 500 to 500 000, containing at least one moiety comprising:
- at least one polyorganosiloxane group, consisting of 1 to 1 000 organosiloxane units in the chain of the moiety or in the form of a graft, and
- at least two groups capable of establishing hydrogen interactions chosen from ester, amide, sulphonamide, carbamate, thiocarbamate, urea, urethane, thiourea, oxamido, guanidino and biguanidino groups, and combinations thereof,
the polymer being solid at room temperature and soluble in the liquid fatty phase at a temperature of 25 to 250°C,
the liquid fatty phase consisting partially or totally of ester oil (s),
and the pigment, the liquid fatty phase and the polymer forming a physiologically acceptable medium.

This make-up composition is preferably self-supporting.

The composition of the invention may be provided in the form of a cake mascara, an eyeliner, a foundation, a lipstick, a blusher, a make-up-removing product, a make-up product for the body, an eyeliner or a face powder, or a concealer product.

The subject of the invention is also a transparent or translucent make-up stick for the skin, the lips and/or the superficial body growths, and in particular for the lips, containing at least one pigment in a sufficient quantity for applying make-up to the skin, the lips and/or the superficial body growths and a liquid continuous fatty phase comprising an ester oil chosen from esters of monocarboxylic acids with monoalcohols and polyalcohols, structured with at least one polymer (homopolymer or copolymer) having a weight-average molecular mass, ranging from 500 to 500 000, containing at least one moiety comprising:
- at least one polyorganosiloxane group, consisting of 1 to 1 000 organosiloxane units in the chain of the moiety or in the form of a graft, and
- at least two groups capable of establishing hydrogen interactions chosen from ester, amide, sulphonamide, carbamate, thiocarbamate, urea, urethane, thiourea, oxamido, guanidino and biguanidino groups, and combinations thereof,
the polymer being solid at room temperature and soluble in the liquid fatty phase at a temperature of 25 to 250°C,
the liquid fatty phase consisting partially or totally of ester oil(s) and of the pigment, the fatty phase and the polymer forming a physiologically acceptable medium.

The invention relates to a cosmetic care, make-up or treatment method for the keratinous materials of human beings, comprising the application to the keratinous materials of a cosmetic composition in accordance with the invention.

The invention also relates to the use of at least one ester oil chosen from the esters of monocarboxylic acids with monoalcohols, in the continuous liquid fatty phase of a cosmetic composition or for the manufacture of a physiologically acceptable composition, the said fatty phase being essentially structured by a sufficient quantity of at least one polymer according to claim 1, said composition being in the form of a transparent anhydrous stick, so as to confer transparent or translucent properties on the said composition and/or on a deposit of the said composition on keratinous materials.

According to an advantageous characteristic of these uses, the composition has a hardeness of 20 to 2 000 gf, preferably of 20 to 900 gf and even better of 20 to 600 gf.

The invention finally relates to a cosmetic method for reducing the transfer and/or the stickiness of a transparent or translucent cosmetic composition in the form of an anhydrous stick containing a liquid fatty phase comprising at least one ester oil chosen from esters of monocarboxylic acids with monoalcohols consisting in structuring the said fatty phase with a sufficient quantity of at least one polymer according to claim 1,
the liquid fatty phase consisting partially or totally of ester oil(s).

The invention will now be described with reference to the following example, given by way of illustration and without limitation.

### Example 1

In this example, the structuring of various oils - among which are ester oils or "short esters" used according to the invention - by a silicone polyamide PASi having a degree of polymerization of 15 (DP15) such as that of Example 3 of Patent US-5 981 680, is studied.

The structuring is studied for two silicone polyamide concentrations of 10 and 25%.

The procedure consists in heating in a small pan, at a temperature of 100 to 105°C or 110°C, a mixture of x% (with x = 10 or 25) of silicone polyamide and the oil to be studied, in pouring the molten mixture into a 30 ml Volga pot, and in observing, after returning to the cold state, the appearance of the poured system.

The characteristics of the compositions studied and the observations made are assembled in the following Table I:

**Table I**

| | PASi concentration | |
|---|---|---|
| Oil | 10% | 25% |
| Parleam | Cloudy liquid | Cloudy separated phase system |
| Octyldodecanol | Translucent gel | Opaque stick |
| Phenylated silicone | Opaque liquid | Opaque stick |
| Isononyl isonanoate | Transparent/ translucent gel | Colourless transparent stick |
| Diisostearyl malate | Cloudy soft gel | Opaque stick |
| Isododecane | Non-homogeneous slightly cloudy liquid | Cloudy, even opaque gel |
| Cyclopentasiloxane | Opaque gel | Opaque hard gel |
| Tridecyl trimellitate | Opaque gel | Opaque gel |
| Caprylic/capric triglyceride | Opaque liquid | Opaque gel |

For the two concentrations used, it is observed that the silicone polyamides lead, after introduction into the various oils and heating, to structuring of most of the oils, that is to say to the formation therein of cast gels or solids, such as sticks.

The observations made and presented in Table 1 clearly demonstrate that among all the oils tested, only isononyl isononanoate, which is an ester oil ("short" ester), in accordance with the invention, makes it possible to obtain not only structured systems, in the form of gels or sticks, but also systems with a transparency or translucence as defined in the present invention, for the various silicone polyamide concentrations studied.

### Example 2

In this example, the structuring of isononyl isononanoate, which is an ester oil in accordance with the invention, is studied using a silicone polyamide similar to that of Example 1, but whose degree of polymerization is higher, namely 30, 45, 75 or 100.

The procedure used is the same as that of Example 1, that is to say that polymers with varying degrees of polymerization at concentrations of 10% to 25% and isononyl isononanoate are mixed, they are heated and they are poured into a pot and the appearance of the poured system is observed after returning to the cold state.

The characteristics of the systems studied and the observations made are assembled in the following Table II:

**Table II**

| | 10% | 25% |
|---|---|---|
| DP15 | Colourless transparent/translucent gel | Colourless transparent/translucent stick |
| DP30 | Colourless transparent gel | Colourless transparent/translucent stick |
| DP45 | Colourless transparent gel | Colourless transparent/translucent stick |
| DP75 | Colourless transparent gel | Colourless transparent/translucent stick |
| DP100 | Colourless transparent gel | Colourless transparent/translucent hard gel |

Table II shows that the structuring and transparent or translucent character (as defined according to the present invention) are not lost when a structured polyamide having a higher degree of polymerization is used.

The examples below demonstrate that the use, in accordance with the invention, of an isononyl isononanoate type oil makes it possible to obtain structured systems based on silicone polyamides having a variable degree of polymerization, alone or as mixtures, which have a translucent or transparent appearance.

### Example 3

In this example, a very simple formula is prepared which leads to a transparent solid system which may be provided in a pot, or even as a. stick, and which may be coloured with colorants, pearlescent agents, glitter, or pigments.

The composition is prepared from the constituents below, according to the procedure of Example 1:
- Silicone polyamide of DP15 20%
- Isononyl isononanoate qs 100

The cast composition obtained in the form of a "stick", with no colouring agent, is transparent for the purposes of the present invention and is colourless.

### Example 4

In this example, in order to demonstrate the non-sticky and transfer-free character upon contact of the formulation according to the invention of Example 3, a stick according to the invention of identical composition is made, but by introducing colour in a concentrated manner, which masks the transparency, but makes it possible to demonstrate the transfer at the optional pressure.

The composition is prepared from the constituents below, according to the procedure of Example 1:
- Silicone polyamide of DP15 20%
- Isononyl isononanoate qs 100
- Pigment 8.66%

The composition when applied for example to the hand is non-sticky. When the deposit is touched with the finger for example by exerting pressure, no colour is removed through contact, that is to say that no colour is transferred to the finger once the contact is terminated.

This example demonstrates that for a composition which is slightly coloured, with colorants for example, it is possible, by virtue of the invention, to obtain the transparency at the same time as the non-stickiness and the absence of transfer through contact.

### REFERENCES

**[1]** EP-A-1 068 856
**[2]** WO-A-01/97758
**[3]** WO-A-97 36573
**[4]** US-A-5 874 069
**[5]** US-A-5 919 441
**[6]** US-A-6 051 216
**[7]** WO-A-02/17870
**[8]** WO-A-02/17871
**[9]** EP-A-1 177 784
**[10]** FR-A-2 817 740
**[11]** FR-A-2 817 739
**[12]** US-A-5 981 680
**[13]** WO-A-99/06473
**[14]** US-A-6 353 076

## Claims

1. Care and/or make-up anhydrous cosmetic composition which is transparent or translucent, optionally capable of giving a transparent or translucent deposit, comprising:
- a liquid fatty phase comprising at least one ester oil chosen from the esters of monocarboxylic acids with monoalcohols, structured with at least one polymer having a weight-average molecular mass ranging from 500 to 500 000, containing at least one moiety of formula (IV) or (V) : 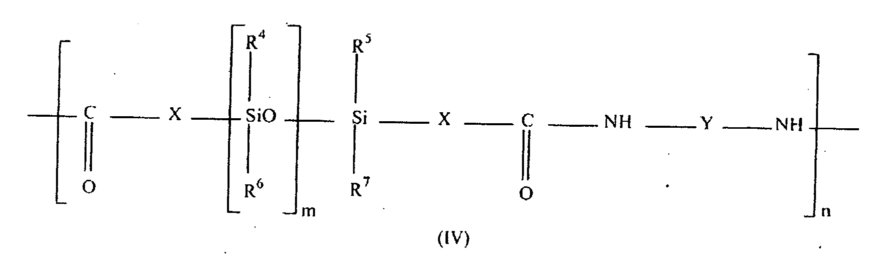 or 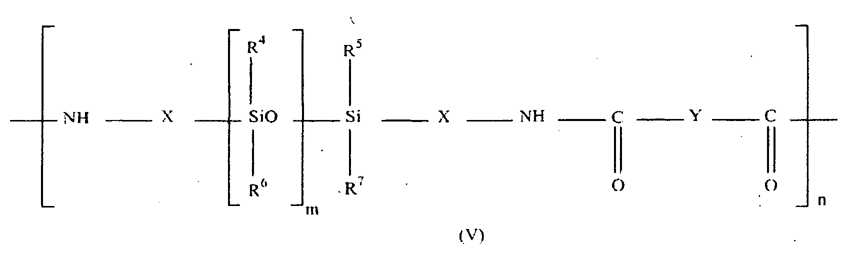 in which 1) R⁴, R⁵, R⁶ and R⁷, which may be identical or different, represent a group chosen from:
- linear, branched or cyclic, saturated or unsaturated, C₁ to C₄₀ hydrocarbon-based groups, possibly containing in their chain one or more oxygen, sulphur and/or nitrogen atoms, and possibly being partially or totally substituted with fluorine atoms,
- C₆ to C₁₀ aryl groups, optionally substituted with one or more C₁ to C₄ alkyl groups,
- polyorganosiloxane chains possibly containing one or more oxygen, sulphur and/or nitrogen atoms;
2) the groups X, which may be identical or different, represent a linear or branched C₁ to C₃₀ alkylenediyl group, possibly containing in its chain one or more oxygen and/or nitrogen atoms;
3) Y is a saturated or unsaturated, C₁ to C₅₀ linear or branched divalent alkylene, arylene, cycloalkylene, alkylarylene or arylalkylene group, possibly comprising one or more oxygen, sulphur and/or nitrogen atoms, and/or bearing as substituent one of the following atoms or groups of atoms: fluorine, hydroxyl, C₃ to C₈ cycloalkyl, C₁ to C₄₀ alkyl, C₅ to C₁₀ aryl, phenyl optionally substituted with 1 to 3 C₁ to C₃ alkyl groups, C₁ to C₃ hydroxyalkyl and C₁ to C₆ aminoalkyl, or
4) Y represents a group corresponding to the formula: 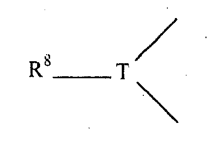 in which
- T represents a linear or branched, saturated or unsaturated, C₃ to C₂₄ trivalent or tetravalent hydrocarbon-based group optionally substituted with a polyorganosiloxane chain, and possibly containing one or more atoms chosen from O, N and S, or T represents a trivalent atom chosen from N, P and Al, and
- R⁸ represents a linear or branched C₁ to C₅₀ alkyl group or a polyorganosiloxane chain, possibly comprising one or more ester, amide, urethane, thiocarbamate, urea, thiourea and/or sulphonamide groups, which may possibly be linked to another chain of the polymer;
5) the groups G, which may be identical or different, represent divalent groups chosen from: 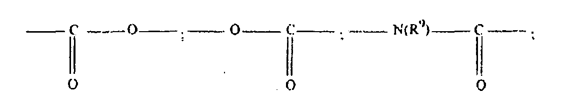 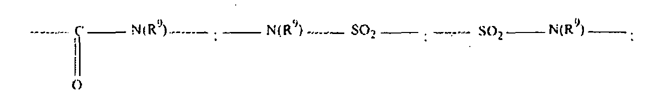 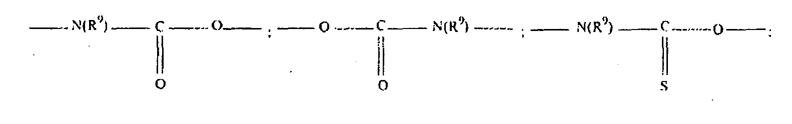 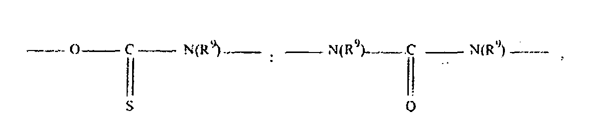 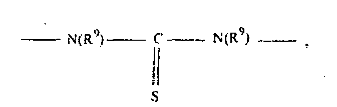 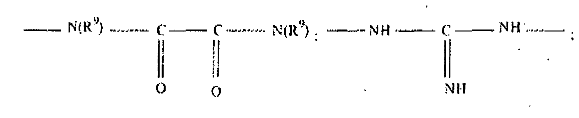 et 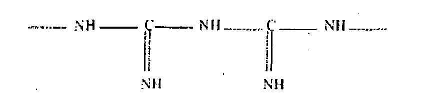 in which R⁹ represents a hydrogen atom or a linear or branched C₁ to C₂₀ alkyl group, on condition that at least 50% of the groups R⁹ of the polymer represent a hydrogen atom and that at least two of the groups G of the polymer are a group other than: 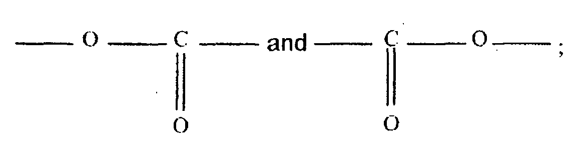
6) n is an integer ranging from 2 to 500 and preferably from 2 to 200, and m is an integer ranging from 1 to 1 000, preferably from 1 to 700 and better still from 6 to 200,
the polymer being solid at room temperature and soluble in the liquid fatty phase at a temperature of 25 to 250°C, and
the liquid fatty phase and the structuring polymer forming a physiologically acceptable medium.

2. Transparent or translucent composition according to Claim 1, in which the said ester corresponds to the following formula (I):
R₁-CO-O-R₂ (I)
where R₁ represents a linear or branched alkyl radical of 1 to 40 carbon atoms, preferably of 7 to 19 carbon atoms, optionally comprising one or more ethylenic double bonds, and optionally substituted; R₂ represents a linear or branched alkyl radical of 1 to 40 carbon atoms, preferably of 3 to 30 carbon atoms and even better of 3 to 20 carbon atoms, optionally comprising one or more ethylenic double bonds, and optionally substituted.

3. Composition according to Claim 2 or 3, in which in formula (I), R₁ represents the residue of a fatty acid comprising from 3 to 40, and even better from 7 to 19 carbon atoms, and R₂ represents a linear or preferably branched hydrocarbon chain containing from 1 to 40, preferably from 3 to 30, and even better from 3 to 20 carbon atoms.

4. Composition according to Claim 2, in which in formula (I), R₁ is a group derived from a fatty acid chosen from the group consisting of acetic, propionic, butyric, caproic, caprylic, pelargonic, capric, undecanoic, lauric, myristic, palmitic, stearic, isostearic, arachidic, behenic, oleic, linolenic, linoleic, oleostearic, arachidonic and erucic acids, and mixtures thereof.

5. Composition according to Claim 4, in which in the formula (I), R₁ is an unsubstituted branched alkyl group of 4 to 14 carbon atoms, preferably of 8 to 10 carbon atoms, and R₂ is an unsubstituted branched alkyl group of 5 to 15 carbon atoms, preferably of 9 to 11 carbon atoms.

6. Composition according to any one of Claims 2 to 5, in which in formula (I), R₁-CO- and R₂ have the same number of carbon atoms and are derived from the same radical.

7. Composition according to any one of Claims 2 to 6, in which in formula (I), the total number of carbon atoms of the groups R₁ and R₂ is greater than or equal to 9.

8. Composition according to any one of Claims 2 to 7, in which the ester oil is chosen from the following compounds:
- isononyl isononanoate,
- isostearyl isostearate,
- and mixtures thereof

9. Composition according to Claim 1, in which the ester oil is chosen from the following compounds:
- cetostearyl octanoate,
- isopropyl myristate,
- 2-ethylhexyl palmitate,
- 2-octyldodecyl stearate,
- 2-octyldodecyl erucate,
- and mixtures thereof.

10. Composition according to any one of the preceding claims, in which the fatty phase comprises from 0.5 to 100% by weight, preferably from 1 to 80%, preferably still from 2 to 50% and even better from 2 to 40% of the ester oil(s).

11. Composition according to any one of Claims 1 to 10, in which the fatty phase contains, as oils, only ester oils excluding any other oil.

12. Composition according to any one of Claims 1 to 11, in which the fatty phase contains only one ester oil which is preferably isononyl isononanoate, preferably in an amount of 100% by weight.

13. Composition according to any one of Claims 1 to 10 and 12, in which the liquid fatty phase comprises at least one silicone oil.

14. Composition according to Claim 1, in which the liquid fatty phase comprises at least one volatile oil having a flash point ranging from 35 to 135°C.

15. Composition according to Claim 1, in which the liquid fatty phase comprises at least one volatile oil having a vapour pressure ranging from 0.01 to 300 mmHg.

16. Composition according to Claim 14 or 15, in which the volatile oil is chosen from isododecane, isohexadecane, C₈-C₁₆ isoparaffins, isohexyl neopentanoate, isodecyl neopentanoate.

17. Composition according to either of Claims 14 and 15, in which the volatile oil is chosen from the group consisting of the following compounds: isododecane, octyltrimethicone, hexyltrimethicone, decamethylcyclopentasiloxane D5, octamethylcyclotetrasiloxane D4, dodecamethylcyclohexasiloxane D6, heptamethyloctyltrisiloxane, decamethyltetrasiloxane, dodecamethylpentasiloxane, polydimethylsiloxane of 1.5 cSt (25°C), polydimethylsiloxane of 2 cSt (25°C), polydimethylsiloxane of 3 cSt (25°C), polydimethylsiloxane of 5 cSt (25°C), and mixtures thereof.

18. Composition according to any one of Claims 14 to 16, in which the volatile oil is chosen from perfluoropolyethers, perfluoroalkanes, perfluoro-adamaritames, esters of perfluoroalkyl phosphates and fluorinated ester oils.

19. Composition according to any one of Claims 14 to 18, in which the liquid fatty phase comprises a nonvolatile silicone oil.

20. Composition according to any one of Claims 13 to 19, in which the liquid fatty phase contains at least 1%, and even better at least 5% by weight, for example from 10 to 90% by weight of silicone oil(s).

21. Composition according to any one of Claims 14 to 20, in which the volatile oil represents from 3 to 89.4%, preferably from 5 to 60%, for example from 5 to 10% of the total weight of the composition.

22. Composition according to any one of the preceding claims, comprising, in addition, solid particles chosen from fillers, pearlescent or non-pearlescent pigments, and mixtures thereof, in a quantity such that the composition remains transparent or translucent.

23. Composition according to Claim 22, in which the particles are pigments chosen from zinc oxides, iron oxides, titanium oxides and mixtures thereof.

24. Composition according to Claim 1, in which X and/or Y represent an alkylene group containing in its alkylene portion at least one of the following elements:
1) 1 to 5 amide, urea, urethane or carbamate groups,
2) a C₅ or C₆ cycloalkyl group, and
3) a phenylene group optionally substituted with 1 to 3 identical or different C₁ to C₃ alkyl groups, and/or substituted with at least one element chosen from the group consisting of:
- a hydroxyl group,
- a C₃ to C₈ cycloalkyl group,
- one to three C₁ to C₄₀ alkyl groups,
- a phenyl group optionally substituted with one to three C₁ to C₃ alkyl groups,
- a C₁ to C₃ hydroxyalkyl group, and
- a C₁ to C₆ aminoalkyl group.

25. Composition according to Claim 1 to 24, in which Y represents: 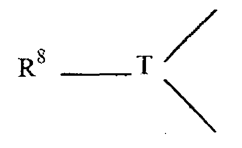 in which R⁸ represents a polyorganosiloxane chain and T represents a group of formula: 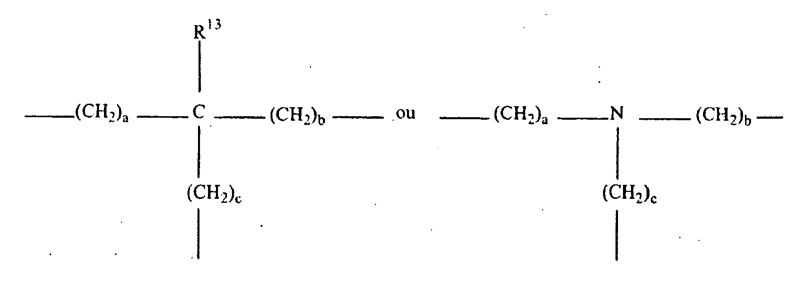 in which a, b and c are, independently, integers ranging from 1 to 10, and R¹³ is a hydrogen atom or a group such as those defined for R⁴, R⁵, R⁶ and R⁷, in Claim 1.

26. Composition according to Claim 1, in which R⁴, R⁵, R⁶ and R⁷ represent, independently, a linear or branched C₁ to C₄₀ alkyl group, preferably a CH₃, C₂H₅, n-C₃H₇ or isopropyl group, a polyorganosiloxane chain or a phenyl group optionally substituted with one to three methyl or ethyl groups.

27. Composition according to any one of the preceding claims, in which the structuring polymer represents from 0.5 to 80% of the total weight of the composition, preferably from 2 to 60% and even better from 5 to 40% of the total weight of the composition.

28. Composition according to any one of the preceding claims, in which the liquid fatty phase further contains a non-silicone oil.

29. Composition according to any one of the preceding claims, in which the liquid fatty phase represents from 5 to 95% of the total weight of the composition and even better from 20 to 75% of the total weight of the composition.

30. Composition according to any one of the preceding claims, **characterized in that** it additionally comprises colouring matter.

31. Composition according to any one of the preceding claims, **characterized in that** it is provided in the form of a transparent or translucent rigid gel, and in particular of a transparent anhydrous stick.

32. Composition according to Claim 27, **characterized in that** it is self-supporting.

33. Composition according to any one of Claims 1 to 32, **characterized in that** it is provided in the form of a cake mascara, an eyeliner, a foundation, a lipstick, a blusher, a make-up-removing product, a make-up product for the body, an eyeshadow or a face powder, or a concealer product.

34. Cosmetic care, make-up or treatment method for the keratinous materials of human beings, comprising the application to the keratinous materials of a cosmetic composition according to one of the preceding claims.

35. Use of at least one ester oil chosen from esters of monocarboxylic acids with monoalcohols, in the continuous liquid fatty phase of a cosmetic composition or for the manufacture of a physiologically acceptable composition, one said fatty phase being essentially structured with a sufficient quantity of at least one polymer according to claim 1, said composition being in the form of a transparent anhydrous stick, to confer properties of tranparency and translucence of the said composition and/or a deposit of the said composition on keratinous materials.

36. Use according to any one of Claims 35, in which the composition has a hardness of 20 to 2 000 gf, preferably of 20 to 900 gf and even better of 20 to 600 gf.

37. Cosmetic method for reducing the transfer and/or stickiness of a transparent or translucent cosmetic composition in the form of an anhydrous stick containing a liquid fatty phase comprising at least one ester oil chosen from the esters of monocarboxylic acids with monoalcohols, consisting in structuring the said fatty phase with a sufficient quantity of at least one polymer according to claim 1,
the liquid fatty phase consisting partially or totally of ester oil(s).

## Patentansprüche

1. Wasserfreie kosmetische Pflege- und/oder Schminkzusammensetzung, die transparent oder transluzid ist und gegebenenfalls befähigt ist, eine transparente oder transluzide Ablagerung zu bilden, enthaltend:
- eine flüssige Fettphase, die mindestens einen als Öl vorliegenden Ester enthält, der unter den Estern von Monocarbonsäuren und Monoalkoholen ausgewählt ist, und die mit mindestens einem Polymer strukturiert ist, das eine gewichtsmittlere Molmasse von 500 bis 500 000 aufweist und zumindest eine Einheit der Formel (IV) oder (V) enthält: 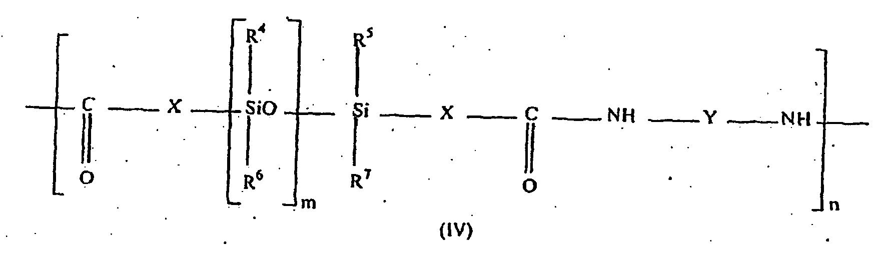 oder 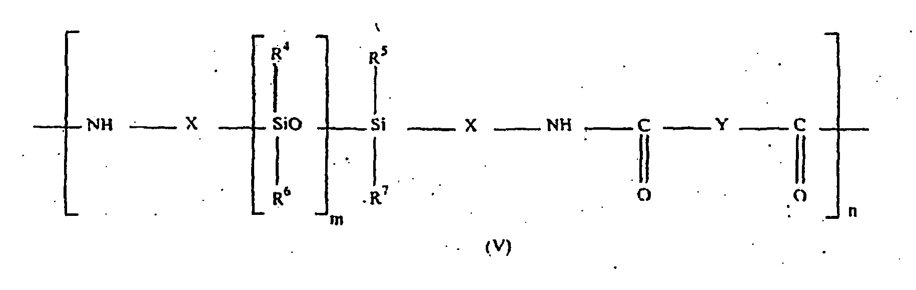
worin
1) R4, R⁵, R6 und R⁷, die gleich oder verschieden sein können, eine Gruppe bedeuten, die ausgewählt ist unter:
- linearen, verzweigten oder cyclischen, gesättigten oder ungesättigten Gruppen auf Kohlenwasserstoffbasis mit 1 bis 40 Kohlenstoffatomen, die gegebenenfalls in ihrer Kette ein oder mehrere Sauerstoff-, Schwefel- und/oder Stickstoffatome enthalten und gegebenenfalls ganz oder teilweise mit Fluoratomen substituiert sind,
- Arylgruppen mit 6 bis 10 Kohlenstoffatomen, die gegebenenfalls mit einer oder mehreren C₁₋₄-Alkylgruppen substituiert sind,
- Polyorganosiloxanketten, die gegebenenfalls ein oder mehrere Sauerstoff-, Schwefel- und/oder Stickstoffatome enthalten;
2) die Gruppen X, die gleich oder verschieden sein können, eine geradkettige oder verzweigte C₁₋₃₀-Alkylendiylgruppe bedeuten, die gegebenenfalls in ihrer Kette ein oder mehrere Sauerstoff- und/oder Stickstoffatome enthält;
3) Y eine gesättigte oder ungesättigte, geradkettige oder verzweigte, zweiwertige Alkylen-, Arylen-, Cycloalkylen-, Alkylarylen- oder Arylalkylengruppe mit 1 bis 50 Kohlenstoffatomen ist, die gegebenenfalls ein oder mehrere Sauerstoff-, Schwefel-und/oder Stickstoffatome enthält und/oder als Substituenten ein oder mehrere der folgenden Atome oder eine oder mehrere der folgenden Gruppen von Atomen enthält: Fluor, Hydroxy, C₃₋₈-Cycloalkyl, C₁₋₄₀-Alkyl, C₅₋₁₀-Aryl, eine Phenylgruppe, die gegebenenfalls mit 1 bis 3 C₁₋₃-Alkylgruppen substituiert ist, C₁₋₃-Hydroxyalkyl und C₁₋₆-Aminoalkyl, oder
4) Y eine Gruppe der folgenden Formel bedeutet: 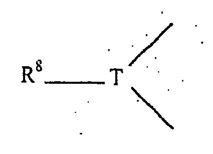 in der
- T eine geradkettige oder verzweigte, gesättigte oder ungesättigte dreiwertige oder vierwertige Gruppe auf Kohlenwasserstoffbasis mit 3 bis 24 Kohlenstoffatomen ist, die gegebenenfalls mit einer Polyorganosiloxankette substituiert ist und gegebenenfalls ein oder mehrere Atome enthält, die unter O, N und S ausgewählt sind, oder T ein dreiwertiges Atom bedeutet, das unter N, P und Al ausgewählt ist, und
- R⁸ eine geradkettige oder verzweigte C₁₋₅₀-Alkylgruppe oder eine Polyorganosiloxankette bedeutet, die gegebenenfalls eine oder mehrere der Gruppen Ester, Amid, Urethan, Thiocarbamat, Harnstoff, Thioharnstoff und/oder Sulfonamid enthält, die gegebenenfalls mit einer anderen Polymerkette verbunden sind;
5) die Gruppen G, die gleich oder verschieden sein können, zweiwertige Gruppen bedeuten, die ausgewählt sind unter: 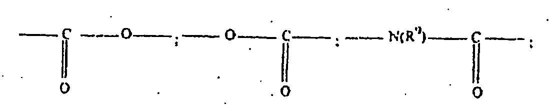 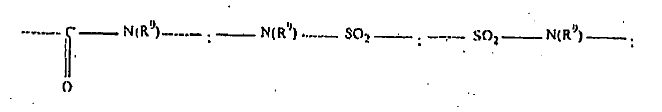 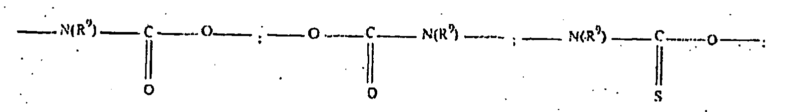 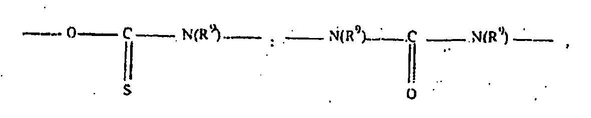 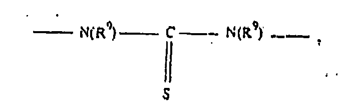 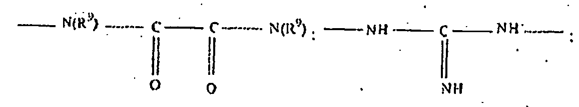 et 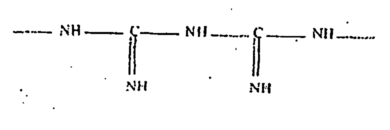 in denen R⁹ ein Wasserstoffatom oder eine geradkettige oder verzweigte C₁₋₂₀-Alkylgruppe bedeutet, mit der Maßgabe, dass mindestens 50 % der Gruppen R₉ des Polymers ein Wasserstoffatom bedeuten und mindestens zwei der Gruppen G des Polymers eine Gruppe bedeuten, die verschieden ist von: 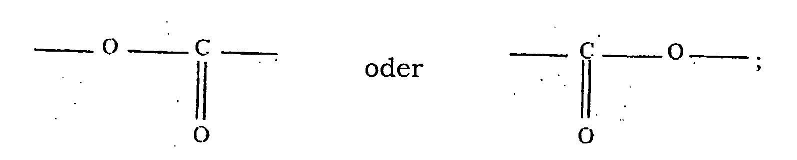
6) n eine ganze Zahl von 2 bis 500 und vorzugsweise 2 bis 200 bedeutet, und m eine ganze Zahl im Bereich von 1 bis 1 000, vorzugsweise 1 bis 700 und noch besser 6 bis 200 ist,
wobei das Polymer bei Raumtemperatur fest ist und in der flüssigen Fettphase bei einer Temperatur von 25 bis 250 °C löslich ist, und
die flüssige Fettphase und das strukturierende Polymer ein physiologisch akzeptables Medium bilden.

2. Transparente oder transluzide Zusammensetzung nach Anspruch 1, in der der Ester der folgenden Formel (I) entspricht:
R₁-CO-O-R₂ (I)
worin R₁ eine geradkettige oder verzweigte Alkylgruppe mit 1 bis 40 Kohlenstoffatomen und vorzugsweise 7 bis 19 Kohlenstoffatomen ist, die gegebenenfalls eine oder mehrere ethylenische Doppelbindungen enthält und gegebenenfalls substituiert ist; R₂ eine geradkettige oder verzweigte Alkylgruppe mit 1 bis 40 Kohlenstoffatomen, vorzugsweise 3 bis 30 Kohlenstoffatomen und besser 3 bis 20 Kohlenstoffatomen ist, die gegebenenfalls eine oder mehrere ethylenische Doppelbindungen enthält und gegebenenfalls substituiert ist.

3. Zusammensetzung nach Anspruch 2 oder 3, wobei in Formel (I) R₁ den Rest einer Fettsäure mit 3 bis 40 Kohlenstoffatomen und besser 7 bis 19 Kohlenstoffatomen bedeutet und R₂ eine geradkettige oder vorzugsweise verzweigte Kohlenwasserstoffkette mit 1 bis 40 Kohlenstoffatomen, vorzugsweise 3 bis 30 Kohlenstoffatomen und besser 3 bis 20 Kohlenstoffatomen bedeutet.

4. Zusammensetzung nach Anspruch 2, wobei in Formel (I) R₁ eine Gruppe bedeutet, die von einer Fettsäure abgeleitet ist, die unter Essigsäure, Propionsäure, Buttersäure, Hexansäure, Caprylsäure, Pelargonsäure, Decansäure, Undecansäure, Laurinsäure, Myristinsäure, Palmitinsäure, Stearinsäure, Isostearinsäure, Arachinsäure, Behensäure, Ölsäure, Linolensäure, Linolsäure, Oleostearinsäure, Arachidonsäure und Erucasäure und deren Gemischen ausgewählt ist.

5. Zusammensetzung nach Anspruch 4, wobei in der Formel (I) R₁ eine nicht substituierte verzweigte Alkylgruppe mit 4 bis 14 Kohlenstoffatomen und vorzugsweise 8 bis 10 Kohlenstoffatomen bedeutet und R₂ eine unsubstituierte Alkylgruppe mit 5 bis 15 Kohlenstoffatomen und vorzugsweise 9 bis 11 Kohlenstoffatomen ist.

6. Zusammensetzung nach einem der Ansprüche 2 bis 5, wobei in Formel (I) R₁-CO- und R₂ die gleiche Anzahl an Kohlenstoffatomen besitzen und von der gleichen Gruppe abgeleitet sind.

7. Zusammensetzung nach einem der Ansprüche 2 bis 6, wobei in Formel (I) die Gesamtzahl der Kohlenstoffatome der Gruppen R₁ und R₂ gleich 9 oder größer als 9 ist.

8. Zusammensetzung nach einem der Ansprüche 2 bis 7, wobei der als Öl vorliegende Ester unter den folgenden Verbindungen ausgewählt ist:
- Isononylisononanoat,
- Isostearylisostearat,
- und deren Gemischen.

9. Zusammensetzung nach Anspruch 1, wobei der als Öl vorliegende Ester unter den folgenden Verbindungen ausgewählt ist:
- Cetostearyloctanoat,
- Isopropylmyristat,
- 2-Ethylhexylpalmitat,
- 2-Octyldodecylstearat,
- 2-Octyldodecylerucat,
- und deren Gemischen.

10. Zusammensetzung nach einem der vorhergehenden Ansprüche, wobei die Fettphase 0,5 bis 100 Gew.-%, vorzugsweise 1 bis 80 %, noch bevorzugter 2 bis 50 % und besser 2 bis 40 % des oder der als Öl vorliegenden Ester(s) enthält.

11. Zusammensetzung nach einem der Ansprüche 1 bis 10, wobei die Fettphase als Öle nur die als Öl vorliegenden Ester und keine weiteren Öle enthält.

12. Zusammensetzung nach einem der Ansprüche 1 bis 11, wobei die Fettphase nur einen als Öl vorliegenden Ester enthält, bei dem es sich vorzugsweise um Isononylisononanoat vorzugsweise in einer Menge von 100 Gew.-% handelt.

13. Zusammensetzung nach einem der Ansprüche 1 bis 10 und 12, wobei die flüssige Fettphase mindestens ein Siliconöl enthält.

14. Zusammensetzung nach Anspruch 1, wobei die flüssige Fettphase mindestens ein flüchtiges Öl mit einem Flammpunkt im Bereich von 35 bis 135 °C enthält.

15. Zusammensetzung nach Anspruch 1, wobei die flüssige Fettphase zumindest ein flüchtiges Öl mit einem Dampfdruck im Bereich von 0,01 bis 300 mmHg enthält.

16. Zusammensetzung nach Anspruch 14 oder 15, wobei das flüchtige Öl unter Isododecan, Isohexadecan, C₈₋₁₆-Isoparaffinen, Isohexylneopentanoat und Isodecylneopentanoat ausgewählt ist.

17. Zusammensetzung nach einem der Ansprüche 14 und 15, wobei das flüchtige Öl unter den folgenden Verbindungen ausgewählt ist: Isododecan, Octyltrimethicon, Hexyltrimethicon, Decamethylcyclopentasiloxan D5, Octamethylcyclotetrasiloxan D4, Dodecamethylcyclohexasiloxan D6, Heptamethyloctyltrisiloxan, Decamethyltetrasiloxan, Dodecamethylpentasiloxan, Polydimethylsiloxan von 1,5 cSt (25 °C), Polydimethylsiloxan von 2 cSt (25 °C), Polydimethylsiloxan von 3 cSt (25 °C), Polydimethylsiloxan von 5 cSt (25 °C) und deren Gemischen.

18. Zusammensetzung nach einem der Ansprüche 14 bis 16, wobei das flüchtige Öl unter den Perfluorpolyethern, Perfluoralkanen, Perfluoradamantanen, Estern von Perfluoralkylphosphaten und fluorierten ölförmigen Estern ausgewählt ist.

19. Zusammensetzung nach einem der Ansprüche 14 bis 18, wobei die flüssige Fettphase ein nichtflüchtiges Siliconöl enthält.

20. Zusammensetzung nach einem der Ansprüche 13 bis 19, wobei die flüssige Fettphase mindestens 1 %, besser mindestens 5 %, beispielsweise 10 bis 90 Gew.-% Siliconöl(e) enthält.

21. Zusammensetzung nach einem der Ansprüche 14 bis 20, wobei das flüchtige Öl 3 bis 89,4 %, vorzugsweise 5 bis 60 % und beispielsweise 5 bis 10 % des Gesamtgewichts der Zusammensetzung ausmacht.

22. Zusammensetzung nach einem der vorhergehenden Ansprüche, die ferner feste Partikel enthält, die unter den Füllstoffen, Perlglanzpigmenten oder Pigmenten und deren Gemischen in einer solchen Menge ausgewählt sind, dass die Zusammensetzung noch transparent oder transluzid bleibt.

23. Zusammensetzung nach Anspruch 22, wobei die Partikel Pigmente sind, die unter den Zinkoxiden, Eisenoxiden, Titanoxiden und deren Gemischen ausgewählt sind.

24. Zusammensetzung nach Anspruch 1, wobei X und/oder Y eine Alkylengruppe bedeuten, die in ihrem Alkylenteil mindestens eines der folgenden Elemente enthält:
1) 1 bis 5 Amid-, Harnstoff-, Urethan- oder Carbamatgruppen,
2) eine C₅- oder C₆-Cycloalkylgruppe, und
3) eine Phenylengruppe, die gegebenenfalls mit 1 bis 3 C₁₋₃-Alkylgruppen, die gleich oder verschieden sind, substituiert ist und/oder substituiert ist mit mindestens einem Element, das ausgewählt ist unter:
- einer Hydroxygruppe,
- einer C₃₋₈-Cycloalkylgruppe,
- 1 bis 3 C₁₋₄-Alkylgruppen,
- einer Phenylgruppe, die gegebenenfalls mit 1 bis 3 C₁₋₃-Alkylgruppen substituiert ist,
- einer C₁₋₃-Hydroxyalkylgruppe, und
- einer C₁₋₆-Aminoalkylgruppe.

25. Zusammensetzung nach Anspruch 1 bis 24, wobei Y bedeutet: 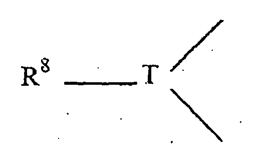 wobei R⁸ eine Polyorganosiloxankette ist und T eine Gruppe der folgenden Formel bedeutet: 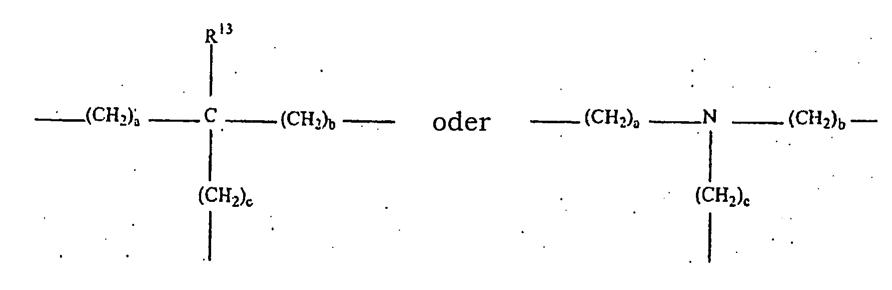 wobei a, b und c unabhängig voneinander ganze Zahlen im Bereich von 1 bis 10 bedeuten und R¹³ ein Wasserstoffatom oder eine Gruppe ist, wie sie für R⁴, R⁵, R⁶ und R⁷ in Anspruch 1 definiert ist.

26. Zusammensetzung nach Anspruch 1, wobei R⁴, R⁵, R6 und R7 unabhängig voneinander eine geradkettige oder verzweigte C₁₋₄₀-Alkylgruppe, vorzugsweise CH₃, C₂H₅, n-C₃H₇ oder Isopropyl, eine Polyorganosiloxankette oder eine Phenylgruppe bedeuten, die gegebenenfalls mit 1 bis 3 Methyl- oder Ethylgruppen substituiert ist.

27. Zusammensetzung nach einem der vorhergehenden Ansprüche, wobei das strukturierende Polymer 0,5 bis 80 % des Gesamtgewichts der Zusammensetzung, vorzugsweise 2 bis 60 % und besser 5 bis 40 % des Gesamtgewichts der Zusammensetzung ausmacht.

28. Zusammensetzung nach einem der vorhergehenden Ansprüche, wobei die flüssige Fettphase ferner ein Öl enthält, bei dem es sich nicht um eine Siliconöl handelt.

29. Zusammensetzung nach einem der vorhergehenden Ansprüche, wobei die flüssige Fettphase 5 bis 95 % des Gesamtgewichts der Zusammensetzung und besser 20 bis 75 % des Gesamtgewichts der Zusammensetzung ausmacht.

30. Zusammensetzung nach einem der vorhergehenden Ansprüche, **dadurch gekennzeichnet, dass** sie ferner Farbmittel enthält.

31. Zusammensetzung nach einem der vorhergehenden Ansprüche, **dadurch gekennzeichnet, dass** sie in Form eines transparenten oder transluziden starren Gels und insbesondere in Form eines transparenten wasserfreien Stifts vorliegt.

32. Zusammensetzung nach Anspruch 27, **dadurch gekennzeichnet, dass** sie selbsttragend ist.

33. Zusammensetzung nach einem der Ansprüche 1 bis 32, **dadurch gekennzeichnet, dass** sie als Mascara-Stein (cake mascara), Eyeliner, Grundierung, Lippenstift, Rouge, Abschminkprodukt, Schminkprodukt für den Körper, Lidschatten oder Gesichtspuder oder als Abdeckprodukt vorliegt.

34. Kosmetisches Verfahren für die Pflege, zum Schminken oder für die Behandlung von menschlichen Keratinsubstanzen, das das Aufbringen einer kosmetischen Zusammensetzung nach einem der vorhergehenden Ansprüche auf die Keratinsubstanzen umfasst.

35. Verwendung mindestens eines ölförmigen Esters, der unter den Ester von Monocarbonsäuren und Monoalkoholen ausgewählt ist, in der kontinuierlichen flüssigen Fettphase einer kosmetischen Zusammensetzung oder für die Herstellung einer physiologisch akzeptablen Zusammensetzung, wobei die Fettphase im Wesentlichen mit einer ausreichenden Menge mindestens eines Polymers nach Anspruch 1 strukturiert ist, wobei die Zusammensetzung in Form eines transparenten wasserfreien Stiftes vorliegt, um der Zusammensetzung und/oder einer Ablagerung dieser Zusammensetzung auf den Keratinsubstanzen die Eigenschaften der Transparenz und Lichtdurchlässigkeit zu geben.

36. Verwendung nach einem der Ansprüche 35, wobei die Zusammensetzung eine Härte von 20 bis 2 000 gf, vorzugsweise 20 bis 900 gf und besser 20 bis 600 gf hat.

37. Kosmetisches Verfahren, um den Transfer und/oder die Klebrigkeit einer transparenten oder transluziden kosmetischen Zusammensetzung in der Form eines wasserfreien Stiftes zu vermindern, die eine flüssige Fettphase mit mindestens einem als Öl vorliegenden Ester enthält, der unter den Estern von Monocarbonsäuren und Monoalkoholen ausgewählt ist, das darin besteht, die Fettphase mit einer ausreichenden Menge mindestens eines Polymers nach Anspruch 1 zu strukturieren, wobei die Fettphase ganz oder teilweise aus einem oder mehreren als Öl vorliegenden Ester(n) besteht.

## Revendications

1. Composition cosmétique anhydre de soins et/ou de maquillage, qui est transparente ou translucide, éventuellement capable de donner lieu à un dépôt transparent ou translucide, comprenant :
- une phase grasse liquide comprenant au moins une huile d'ester choisie parmi les esters d'acides monocarboxyliques avec des mono-alcools, structurés avec au moins un polymère présentant une masse moléculaire moyenne en poids s'échelonnant de 500 à 500 000, renfermant au moins un fragment de formules (IV) ou (V) : 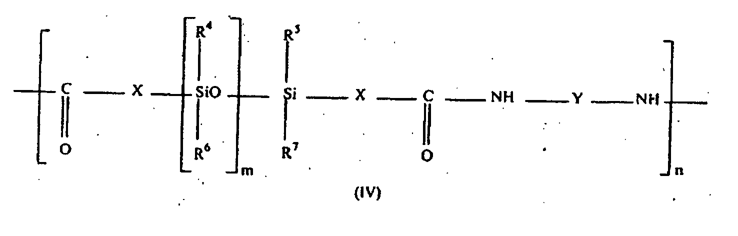
ou 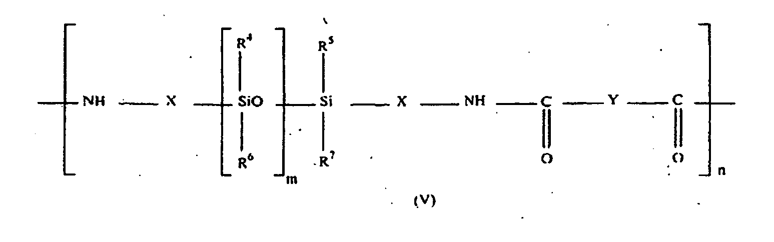 dans lesquelles 1) R⁴, R⁵ R⁶ et R⁷, qui peuvent être identiques ou différents, représentent un groupe choisi parmi :
- des groupes à base hydrocarbonée en C₁ à C₄₀, linéaires, ramifiés ou cycliques, saturés ou insaturés, renfermant éventuellement dans leur chaîne un ou plusieurs atomes d'oxygène, de soufre et/ou d'azote, et étant éventuellement partiellement ou totalement substitués par des atomes de fluor,
- des groupes aryle en C₆ à C₁₀, éventuellement substitués par un ou plusieurs groupes alkyle en C₁ à C₄,
- des chaînes de polyorganosiloxane renfermant éventuellement un ou plusieurs atomes d'oxygène, de soufre et/ou d'azote ;
2) les groupes X, qui peuvent être identiques ou différents, représentent un groupe alkylènediyle en C₁ à C₃₀, linéaire ou ramifié, renfermant éventuellement dans sa chaîne un ou plusieurs atomes d'oxygène et/ou d'azote ;
3) Y est un groupe bivalent en C₁ à C₅₀, linéaire ou ramifié, saturé ou insaturé, alkylène, arylène, cycloalkylène, alkylarylène ou arylalkylène, renfermant éventuellement un ou plusieurs atomes d'oxygène, de soufre et/ou d'azote, et/ou portant, en tant que substituant, l'un des atomes ou groupes d'atomes suivants : un atome de fluor, un groupe hydroxyle, un groupe cycloalkyle en C₃ à C₈, un groupe alkyle C₁ à C₄₀, un groupe aryle en C₅ à C₁₀, un groupe phényle éventuellement substitué par de 1 à 3 groupes alkyle en C₁ à C₃, un groupe hydroxyalkyle en C₁ à C₃ et un groupe aminoalkyle en C₁ à C₆, ou
4) Y représente un groupe correspondant à la formule : 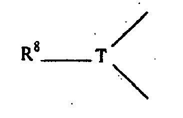 dans laquelle
- T représente un groupe à base hydrocarbonée en C₃ à C₂₄, trivalent ou tétravalent, linéaire ou ramifié, saturé ou insaturé, éventuellement substitué par une chaîne de polyorganosiloxane, et renfermant éventuellement un ou plusieurs atomes choisis parmi un atome d'oxygène, un atome d'azote et un atome de soufre, ou T représente un atome trivalent choisi parmi un atome d'azote, un atome de phosphore et un atome d'aluminium, et
- R⁸ représente un groupe alkyle en C₁ à C₅₀, linéaire ou ramifié, ou une chaîne de polyorganosiloxane, renfermant éventuellement un ou plusieurs groupes ester, amide, uréthane, thiocarbamate, urée, thio-urée et/ou sulfonamide, qui peut éventuellement être lié à une autre chaîne du polymère ;
5) les groupes G, qui peuvent être identiques ou différents, représentent des groupes bivalents choisis parmi : 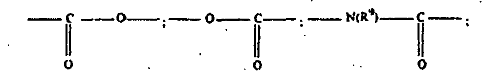 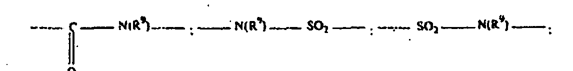 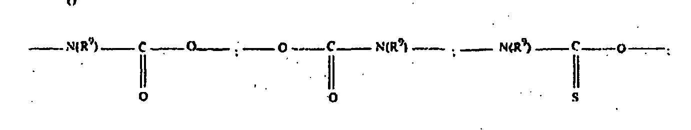 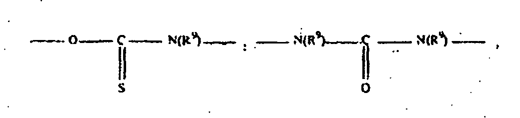 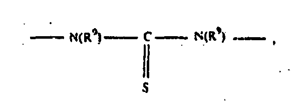 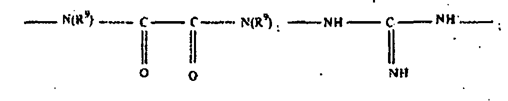 et 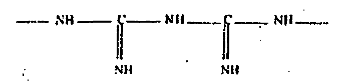 dans lesquels R⁹ représente un atome d'hydrogène ou un groupe alkyle en C₁ à C₂₀, linéaire ou ramifié, à condition qu'au moins 50 % des groupes R⁹ du polymère représentent un atome d'hydrogène et qu'au moins deux des groupes G du polymère soient un groupe autre que : 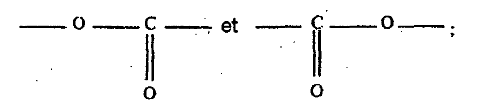
6) n est un entier s'échelonnant de 2 à 500, et de préférence de 2 à 200, et m est un entier s'échelonnant de 1 à 1 000, de préférence de 1 à 700, et encore mieux de 6 à 200,
le polymère étant solide à la température ambiante et soluble dans la phase grasse liquide à une température de 25 à 250°C, et
la phase grasse liquide et le polymère de structuration formant un milieu physiologiquement acceptable.

2. Composition transparente ou translucide selon la revendication 1, dans laquelle ledit ester correspond à la formule (I) suivante :
R₁-CO-O-R₂ (I)
dans laquelle R₁ représente un radical alkyle linéaire ou ramifié renfermant de 1 à 40 atomes de carbone, de préférence de 7 à 19 atomes de carbone, renfermant éventuellement une ou plusieurs doubles liaisons éthyléniques, et éventuellement substitué ;
R₂ représente un radical alkyle linéaire ou ramifié renfermant de 1 à 40 atomes de carbone, de préférence de 3 à 30 atomes de carbone et encore mieux de 3 à 20 atomes de carbone, renfermant éventuellement une ou plusieurs doubles liaisons éthyléniques, et éventuellement substitué.

3. Composition selon la revendication 2 ou 3, dans laquelle dans la formule (I), R₁ représente le résidu d'un acide gras renfermant de 3 à 40, et encore mieux de 7 à 19 atomes de carbone, et R₂ représente une chaîne hydrocarbonée linéaire ou de préférence ramifiée, renfermant de 1 à 40, de préférence de 3 à 30, et encore mieux de 3 à 20 atomes de carbone.

4. Composition selon la revendication 2, dans laquelle dans la formule (I), R₁ est un groupe dérivé d'un acide gras choisi parmi le groupe constitué de l'acide acétique, de l'acide propionique, de l'acide butyrique, de l'acide caproïque, de l'acide caprylique, de l'acide pelargonique, de l'acide caprique, de l'acide undécanoïque, de l'acide laurique, de l'acide myristique, de l'acide palmitique, de l'acide stéarique, de l'acide isostéarique, de l'acide arachidique, de l'acide béhénique, de l'acide oléique, de l'acide linolénique, de l'acide linoléique, de l'acide oléostéarique, de l'acide arachidonique et de l'acide érucique, et de leurs mélanges.

5. Composition selon la revendication 4, dans laquelle dans la formule (I), R₁ est un groupe alkyle ramifié non substitué renfermant de 4 à 14 atomes de carbone, de préférence de 8 à 10 atomes de carbone, et R₂ est un groupe alkyle ramifié non substitué renfermant de 5 à 15 atomes de carbone, de préférence de 9 à 11 atomes de carbone.

6. Composition selon l'une quelconque des revendications 2 à 5, dans laquelle dans la formule (I), R₁-CO- et R₂ présentent le même nombre d'atomes de carbone et sont dérivés du même radical.

7. Composition selon l'une quelconque des revendications 2 à 6, dans laquelle dans la formule (I), le nombre total d'atomes de carbone des groupes R₁ et R₂ est supérieur ou égal à 9.

8. Composition selon l'une quelconque des revendications 2 à 7, dans laquelle l'huile d'ester est choisie parmi les composés suivants :
- l'isononanoate d'isononyle,
- l'isostéarate d'isostéaryle,
- et leurs mélanges.

9. Composition selon la revendication 1, dans laquelle l'huile d'ester est choisie parmi les composés suivants :
- l'octanoate de cétostéaryle,
- le myristate d'isopropyle,
- le palmitate de 2-éthylhexyle,
- le stéarate de 2-octyldodécyle,
- l'érucate de 2-octyldodécyle,
- et leurs mélanges.

10. Composition selon l'une quelconque des revendications précédentes, dans laquelle la phase grasse comprend de 0,5 à 100 % en poids, de préférence de 1 à 80 %, encore plus préférablement de 2 à 50 %, et encore mieux de 2 à 40 % de l'huile (des huiles) d'ester.

11. Composition selon l'une quelconque des revendications 1 à 10, dans laquelle la phase grasse contient, en tant que huiles, seulement des huiles d'ester à l'exclusion de toute autre huile.

12. Composition selon l'une quelconque des revendications 1 à 11, dans laquelle la phase grasse contient seulement une huile d'ester qui est de préférence l'isononanoate d'isononyle, de préférence en une quantité de 100 % en poids.

13. Composition selon l'une quelconque des revendications 1 à 10 et 12, dans laquelle la phase grasse liquide comprend au moins une huile de silicone.

14. Composition selon la revendication 1, dans laquelle la phase grasse liquide comprend au moins une huile volatile présentant un point éclair s'échelonnant de 35 à 135°C.

15. Composition selon la revendication 1, dans laquelle la phase grasse liquide comprend au moins une huile volatile présentant une pression de vapeur s'échelonnant de 0,01 à 300 mm Hg.

16. Composition selon la revendication 14 ou 15, dans laquelle l'huile volatile est choisie parmi l'isododécane, l'isohexadécane, des isoparaffines en C₈-C₁₆, le néopentanoate d'isohexyle, le néopentanoate d'isodécyle.

17. Composition selon l'une ou l'autre des revendications 14 et 15, dans laquelle l'huile volatile est choisie parmi le groupe constitué des composés suivants : l'isododécane, l'octyltriméthicone, l'hexyltriméthicone, le décaméthylcyclopentasiloxane D5, l'octaméthylcyclotétrasiloxane D4, le dodécaméthylcyclohexasiloxane D6, l'heptaméthyloctyltrisiloxane, le décaméthyltétrasiloxane, le dodécaméthylpentasiloxane, un polydiméthylsiloxane de 1,5 cSt (25°C), un polydiméthylsiloxane de 2 cSt (25°C), un polydiméthylsiloxane de 3 cSt (25°C), un polydiméthylsiloxane de 5 cSt (25°C), et leurs mélanges.

18. Composition selon l'une quelconque des revendications 14 à 16, dans laquelle l'huile volatile est choisie parmi des perfluoropolyéthers, des perfluoroalcanes, des perfluoroadamantanes, des esters de phosphates de perfluoroalkyle et des huiles d'esters fluorés.

19. Composition selon l'une quelconque des revendications 14 à 18, dans laquelle la phase grasse liquide comprend une huile de silicone non volatile.

20. Composition selon l'une quelconque des revendications 13 à 19, dans laquelle la phase grasse liquide contient au moins 1 %, et encore mieux au moins 5 % en poids, par exemple de 10 à 90 % en poids d'huile(s) de silicone.

21. Composition selon l'une quelconque des revendications 14 à 20, dans laquelle l'huile volatile représente de 3 à 89,4 %, de préférence de 5 à 60 %, par exemple de 5 à 10 % du poids total de la composition.

22. Composition selon l'une quelconque des revendications précédentes, comprenant en outre des particules solides choisies parmi des charges, des pigments nacrés ou non nacrés et leurs mélanges, en une quantité telle que la composition demeure transparente ou translucide.

23. Composition selon la revendication 22, dans laquelle les particules sont des pigments choisis parmi des oxydes de zinc, des oxydes de fer, des oxydes de titane et leurs mélanges.

24. Composition selon la revendication 1, dans laquelle X et/ou Y représentent un groupe alkylène renfermant dans sa portion alkylène au moins l'un des éléments suivants :
1) de 1 à 5 groupes amide, urée, uréthane ou carbamate,
2) un groupe cycloalkyle en C₅ ou C₆, et
3) un groupe phénylène éventuellement substitué par de 1 à 3 groupes alkyle en C₁ à C₃, identiques ou différents, et/ou substitué par au moins un élément choisi parmi le groupe constitué :
- d'un groupe hydroxyle,
- d'un groupe cycloalkyle en C₃ à C₈,
- de un à trois groupes alkyle en C₁ à C₄₀,
- d'un groupe phényle éventuellement substitué par de un à trois groupes alkyle en C₁ à C₃,
- d'un groupe hydroxyalkyle en C₁ à C₃, et
- d'un groupe aminoalkyle en C₁ à C₆.

25. Composition selon les revendications 1 à 24, dans laquelle Y représente : 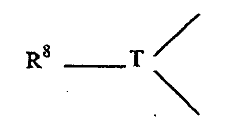 dans laquelle R⁸ représente une chaîne de polyorganosiloxane et T représente un groupe de formules : 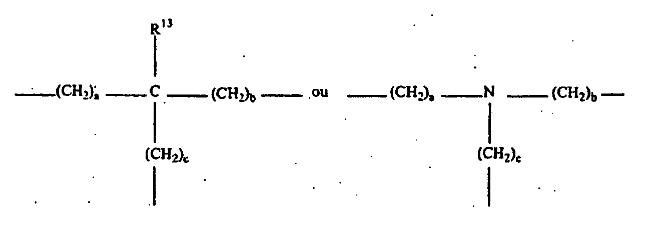 dans lesquelles a, b et c sont indépendamment des entiers s'échelonnant de 1 à 10, et R¹³ est un atome d'hydrogène ou un groupe tel que ceux définis pour R⁴, R⁵, R⁶ et R⁷, dans la revendication 1.

26. Composition selon la revendication 1, dans laquelle R⁴, R⁵ R⁶ et R⁷ représentent indépendamment un groupe alkyle en C₁ à C₄₀, linéaire ou ramifié, de préférence un groupe CH₃, C₂H₅, n-C₃H₇ ou isopropyle, une chaîne de polyorganosiloxane ou un groupe phényle éventuellement substitué par de un à trois groupes méthyle ou éthyle.

27. Composition selon l'une quelconque des revendications précédentes, dans laquelle le polymère de structuration représente de 0,5 à 80 % du poids total de la composition, de préférence de 2 à 60 % et encore mieux de 5 à 40 % du poids total de la composition.

28. Composition selon l'une quelconque des revendications précédentes, dans laquelle la phase grasse liquide contient en outre une huile non siliconée.

29. Composition selon l'une quelconque des revendications précédentes, dans laquelle la phase grasse liquide représente de 5 à 95 % du poids total de la composition et encore mieux de 20 à 75 % du poids total de la composition.

30. Composition selon l'une quelconque des revendications précédentes, **caractérisée en ce qu'**elle comprend en outre une matière colorée.

31. Composition selon l'une quelconque des revendications précédentes, **caractérisée en ce qu'**elle est procurée sous la forme d'un gel rigide transparent ou translucide, et en particulier d'un bâtonnet anhydre transparent.

32. Composition selon la revendication 27, **caractérisée en ce qu'**elle est autoportante.

33. Composition selon l'une quelconque des revendications 1 à 32, **caractérisée en ce qu'** elle est procurée sous la forme d'un cake mascara, d'un ligneur, d'un fond de teint, d'un rouge à lèvres, d'un fard à jupes, d'un produit démaquillant, d'un produit de maquillage pour le corps, d'un fard à paupières ou d'une poudre pour le visage, ou d'un produit correcteur.

34. Méthode de soin cosmétique, de maquillage ou de traitement pour les matières kératineuses d'êtres humains, comprenant l'application, sur les matières kératineuses, d'une composition cosmétique selon l'une des revendications précédentes.

35. Utilisation d'au moins une huile d'ester choisie parmi des esters d'acides monocarboxyliques avec des mono-alcools, dans la phase grasse liquide continue d'une composition cosmétique ou pour la fabrication d'une composition physiologiquement acceptable, une dite phase grasse étant essentiellement structurée avec une quantité suffisante d'au moins un polymère selon la revendication 1, ladite composition étant sous la forme d'un bâtonnet anhydre transparent, pour conférer des propriétés de transparence et de translucidité à ladite composition et/ou d'un dépôt de ladite composition sur des matières kératineuses.

36. Utilisation selon l'une quelconque des revendications 35, dans laquelle la composition présente une dureté de 20 à 2 000 gf, de préférence de 20 à 900 gf et encore mieux de 20 à 600 gf.

37. Méthode cosmétique pour la réduction du transfert et/ou du caractère collant d'une composition cosmétique transparente ou translucide sous la forme d'un bâtonnet anhydre contenant une phase grasse liquide comprenant au moins une huile d'ester choisie parmi les esters d'acides monocarboxyliques avec des mono-alcools, consistant à structurer ladite phase grasse avec une quantité suffisante d'au moins un polymère selon la revendication 1,
la phase grasse liquide étant constituée partiellement ou totalement d'une huile (d'huiles) d'ester.
